# EUROPEAN PATENT SPECIFICATION

(11) **EP 2 203 570 B1**
(45) Date of publication and mention of the grant of the patent: **23.04.2014**
(21) Application number: 07815900.1
(22) Date of filing: 26.09.2007
(51) Int. Cl.: C12Q 1/68

(54) **3.4 KB MITOCHONDRIAL DNA DELETION FOR USE IN THE DETECTION OF CANCER**
3,4 KB GROSSE MITOCHONDRIEN-DNA-DELETION ZUR VERWENDUNG BEIM KREBSNACHWEIS
DÉLÉTION D'ADN MITOCHONDRIAL DE 3,4 KB POUR UTILISATION DANS LA DÉTECTION DU CANCER

(43) Date of publication of application: 07.07.2010
(60) Divisional application: 13169334.3
(73) Proprietor: Mitomics Inc., Thunder Bay, ON P7A 7T1 (CA)
(72) Inventor: PARR, Ryan, Thunder Bay, Ontario P7G 1J5 (CA); THAYER, Robert, Thunder Bay, Ontario P7A 3T6 (CA); DAKUBO, Gabriel, Thunder Bay, Ontario P7J 1H7 (CA); CREED, Jennifer, Thunder Bay, Ontario P7A 7Z6 (CA); ROBINSON, Kerry, Thunder Bay, Ontario P7A 7T1 (CA); MAGGRAH, Andrea, Thunder Bay, Ontario P7A 7S7 (CA); REGULY, Brian, Thunder Bay, Ontario P7A 5S9 (CA)
(74) Representative: UEXKÜLL & STOLBERG
(86) International application number: PCT/CA2007/001711
(87) International publication number: WO 2009/039601

(56) References cited:
- WO-A1-2006/111029
- US-A1- 2005 026 167
- YE CHUANZHONG ET AL: "Quantitative analysis of mitochondrial DNA 4977-bp deletion in sporadic breast cancer and benign breast diseases" BREAST CANCER RESEARCH AND TREATMENT, vol. 108, no. 3, 31 May 2007 (2007-05-31), pages 427-434, XP002591332 ISSN: 0167-6806
- TAN DUAN-JUN ET AL: "Comprehensive scanning of somatic mitochondrial DNA mutations in breast cancer" CANCER RESEARCH, vol. 62, no. 4, 15 February 2002 (2002-02-15), pages 972-976, XP002591333 ISSN: 0008-5472
- PARRELLA PAOLA ET AL: "Detection of mitochondrial DNA mutations in primary breast cancer and fine-needle aspirates" CANCER RESEARCH, AMERICAN ASSOCIATION FOR CANCER REREARCH, US, vol. 61, no. 20, 15 October 2001 (2001-10-15), pages 7623-7626, XP002244378 ISSN: 0008-5472
- ZHU, W. ET AL.: 'Large-scale mitochondrial DNA deletion mutations and nuclear genome instability in human breast cancer' CANCER DETECTION AND PREVENTION vol. 28, no. 2, 2004, pages 119 - 126, XP002524596
- HE, L. ET AL.: 'Detection and quantification of mitochondrial DNA deletions in individual cells by real-time PCR' NUCLEIC ACIDS RESEARCH, [Online] vol. 30, no. 14, 15 July 2002, page E68, XP002372964 ISSN: 1362-4962 Retrieved from the Internet: <URL:http://nar.oxfordjournals.org/cgi/repr int/30/14/e68> [retrieved on 2008-03-04]
- MAKI, J. ET AL.: 'Mitochondrial genome deletion aids in the identification of false- and true-negative prostate needle core biopsy specimens' AMERICAN JOURNAL OF CLINICAL PATHOLOGY vol. 129, no. 1, January 2008, pages 57 - 66, XP008132881
- ANDERSON S ET AL: "Sequence and organization of the human mitochondrial genome.", NATURE 9 APR 1981, vol. 290, no. 5806, 9 April 1981 (1981-04-09), pages 457-465, ISSN: 0028-0836

## Description

### CROSS REFERENCE TO RELATED APPLICATIONS

This application is a continuation-in-part of PCT application no. PCT/CA2006/000652 filed on April 18, 2006 which claims priority from U.S. provisional application nos. 60/672,016 filed April 18, 2005, 60/721,522 filed September 29, 2005, and 60/789,872 filed April 7, 2006.

### FIELD OF THE INVENTION:

This invention is related to the field of mitochondrial genomics. In particular it is related to a 3.4 kb deletion in the mitochondrial genome and its utility as an indicator of cancer.

### DESCRIPTION OF THE PRIOR ART

### Mitochondrial DNA (MtDNA) as a Diagnostic Tool

MtDNA sequence dynamics are important diagnostic tools. Mutations in mtDNA are often preliminary indicators of developing disease, often associated with nuclear mutations, and act as biomarkers specifically related to: disease, such as but not limited to, tissue damage and cancer from smoking and exposure to second hand tobacco smoke (Lee et al., 1998; Wei, 1998); longevity, based on accumulation of mitochondrial genome mutations beginning around 20 years of age and increasing thereafter (von Wurmb, 1998); metastatic disease caused by mutation or exposure to carcinogens, mutagens, ultraviolet radiation (Birch-Machin, 2000); osteoarthritis; cardiovascular, Alzheimer, Parkinson disease (Shoffner et al., 1993; Sherratt et al., 1997;Zhang et al, 1998); age associated hearing loss (Seidman et al., 1997); optic nerve degeneration and cardiac dysrhythmia (Brown et al., 1997; Wallace et al., 1988); chronic progressive external exophthalmoplegia (Taniike et al., 1992); atherosclerosis (Bogliolo et al., 1999); papillary thyroid carcinomas and thyroid tumours (Yeh et al., 2000); as well as others (e.g. Naviaux, 1997; Chinnery and Turnbull, 1999).

Mutations at specific sites of the mitochondrial genome can be associated with certain diseases. For example, mutations at positions 4216, 4217 and 4917 are associated with Leber's Hereditary Optic Neuropathy (LHON) (Mitochondrial Research Society; Huoponen (2001); MitoMap). A mutation at 15452 was found in 5/5 patients to be associated with ubiquinol cytochrome c reductase (complex III) deficiency (Valnot et al. 1999).

Specifically, these mutations or alterations include point mutations (transitions, transversions), deletions (one base to thousands of bases), inversions, duplications, (one base to thousands of bases), recombinations and insertions (one base to thousands of bases). In addition, specific base pair alterations, deletions, or combinations thereof have been found to be associated with early onset of prostate, skin, and lung cancer, as well as aging (e.g. Polyak et al., 1998), premature aging, exposure to carcinogens (Lee et al., 1998), etc.

### Prostate Cancer

Prostate cancer is a frequently diagnosed solid tumour that most likely originates in the prostate epithelium (Huang et al. 1999). In 1997, nearly 10 million American men were screened for prostate specific antigen (PSA), the presence of which suggests prostate cancer (Woodwell, 1999). Indeed, this indicates an even higher number of men screened by an initial digital rectal exam (DRE). In the same year, 31 million men had a DRE (Woodwell, 1999). Moreover, the annual number of newly diagnosed cases of prostate cancer in the United States is estimated at 179,000 (Landis et al., 1999). It is the second most commonly diagnosed cancer and second leading cause of cancer mortality in Canadian men. In 1997 prostate cancer accounted for 19,800 of newly diagnosed cancers in Canadian men (28%) (National Cancer Institute of Canada). It is estimated that 30% to 40% of all men over the age of forty-nine (49) have some cancerous prostate cells, yet only 20% to 25% of these men have a clinically significant form of prostate cancer (SpringNet - CE Connection, internet, www.springnet.com/ce/j803a.htm). Prostate cancer exhibits a wide variety of histological behaviour involving both endogenous and exogenous factors, i.e. socio-economic situations, diet, geography, hormonal imbalance, family history and genetic constitution (Konishi et al. 1997; Hayward et al. 1998). Although certain mtDNA alterations have been previously associated with prostate cancer, the need exists for further markers for the detection of prostate cancer.

### 3.4kb mtDNA deletion and the detection of prostate cancer.

In the applicant's pending PCT application bearing publication no. WO/06/111029 a deletion of a 3379 bp segment of mtDNA was identified through full mitochondrial genome amplification of prostate tissue. The 3379 bp deletion (referred to as the 3.4 kb deletion) was determined to be located between nucleotides 10744-14124 of the mitochondrial genome. It was determined that the detection of this deletion could be used in the diagnosis of prostrate cancer when tissue samples are tested. WO 2006/111029 does not teach the use of mtDNA having the subject deletion in the detection of cancers other than prostate cancer.

The 3.4 kb deletion removes all or part of the following genes from the mtDNA genome: (i) NADH dehydrogenase subunit 4L, (ii) NADH dehydrogenase subunit 4, (iii) NADH dehydrogenase subunit 5, (iv) tRNA histidine, (v) tRNA serine2, and (vi) tRNA leucine2.

### Breast Cancer

Breast cancer is a cancer of the glandular breast tissue and is the fifth most common cause of cancer death. In 2005, breast cancer caused 502,000 deaths (7% of cancer deaths; almost 1% of all deaths) worldwide (World Health Organization Cancer Fact Sheet No. 297). Among women worldwide, breast cancer is the most common cancer and the most common cause of cancer death (World Health Organization Cancer Fact Sheet No. 297). Deletions and mutations of the mitochondrial genome in breast cancer were investigated in Zhu et al. (2004, Cancer detection and prevention, 28(2):119-26), Tan Duan-Jun et al. (2002, Cancer Research, 62(4):972-6) and Parella et al. (2001, Cancer Research, 61(20):7623-6). For example, Zhu et al. reports a relationship between breast cancer and a mtDNA comprising a 4576 bp deletion within a 4977 bp so-called common deletion. The publication also discloses that two other deletions within this common deletion, the 3938 bp and 4388 bp deletions, were not found to correlate with breast cancer. None of these documents discloses a deletion of approximately 3379 bp as a marker for breast cancer. Although certain mtDNA alterations have been previously associated with breast cancer, for example in Parrella et al. (Cancer Research: 61, 2001), the need exists for further markers for the detection of breast cancer.

### SUMMARY OF THE INVENTION

In one embodiment, the present invention provides a method of detecting a cancer in an individual comprising:
a) extracting mitochondrial DNA, mtDNA, from a biological sample from the individual;
b) quantifying the amount of mtDNA in the sample having a deletion of approximately 3379 base pairs in the nucleic acid sequence spanning approximately residues 10744 to 14124 of the mtDNA genome;
c) comparing the amount of mtDNA in the sample having the deletion to at least one known reference value;
wherein the cancer is breast cancer.

In one embodiment, the at least one known reference value is the amount of the deletion in a reference sample of mtDNA from known non-cancerous tissue or body fluid.

In a further embodiment, the at least one known reference value is the amount of the deletion in a reference sample of mtDNA from known cancerous tissue or body fluid.

In another embodiment, the known reference value is the amount of the deletion in a reference sample of mtDNA from known non-cancerous tissue or body fluid, wherein an elevated amount of the deletion in the biological sample compared to the reference sample is indicative of cancer. In this embodiment, the method may further comprise the step of comparing the amount of mtDNA in the sample having the deletion to the amount of the deletion in a reference sample of mtDNA from known cancerous tissue or bod fluid.

In one embodiment, the known reference value is the amount of the deletion in a reference sample of mtDNA from known cancerous tissue or body fluid, wherein a similar level of the deletion in the biological sample compared to the reference sample is indicative of cancer. In this embodiment, the method may further comprise the step of comparing the amount of mtDNA in the sample having the deletion to the amount of the deletion in a reference sample of mtDNA from known non-cancerous tissue or body fluid.

The present invention further provides a method of monitoring an individual for the development of a cancer comprising:
a) extracting mtDNA from a biological sample from the individual;
b) quantifying the amount of mtDNA in the sample having a deletion of approximately 3379 base pairs in the nucleic acid sequence spanning approximately residues 10744 to 14124 of the mtDNA genome;
c) repeating steps a) to b) over a duration of time,
wherein an increasing level of the deletion over the duration of time is indicative of cancer; and wherein the cancer is breast cancer.

In one embodiment, this method further comprises at least one step selected from the group consisting of: (a) comparing the amount of mtDNA in the sample having the deletion to the amount of the deletion in a reference sample of mtDNA from known non-cancerous tissue or body fluid; and (b) comparing the amount of mtDNA in the sample having the deletion to the amount of the deletion in a reference sample of mtDNA from known cancerous tissue or body fluid.

In further embodiments, the deletion has a nucleic acid sequence corresponding to the sequence identified in SEQ ID NO: 1 in the methods of the present invention.

In other embodiments, the quantifying of the deletion in the methods of the present invention includes first amplifying a target region of mtDNA that is indicative of the deletion, and quantifying the amount of the amplified target region. In these embodiments, the amplifying of the target region can be conducted using a pair of amplification primers, one of the pair of amplification primers overlapping a splice joining regions on opposite ends of the deletion.

In further embodiments, the step of quantifying is conducted using real-time PCR in the methods of the present invention.

In one embodiment of the invention, the step of quantifying is conducted using real-time PCR and the reference value is a cycle threshold.

In other embodiments a PCR primer having a sequence corresponding to SEQ ID NO: 2 is used as part of a pair of amplification primers for amplifying the target region.

In further embodiments, one of a pair of PCR primers used in the amplifying of the target region overlaps a spliced region of mtDNA after deletion of the sequence corresponding to SEO ID NO: 1.

In further embodiments, the biological sample is a body tissue or body fluid. In other embodiments, the biological sample is selected from breast tissue and urine.

### BRIEF DESCRIPTION OF THE DRAWINGS

An embodiment of the invention will now be described by way of example only with reference to the appended drawings wherein:
Figure 1 is a schematic diagram showing the design and sequence of a primer useful for the detection of the 3.4 kb deletion.
Figure 2 is a graph showing a comparison of cycle threshold between malignant and symptomatic benign participants in the 3.4 kb study.
Figure 3 is a graph showing cycle threshold as related to Example 1.

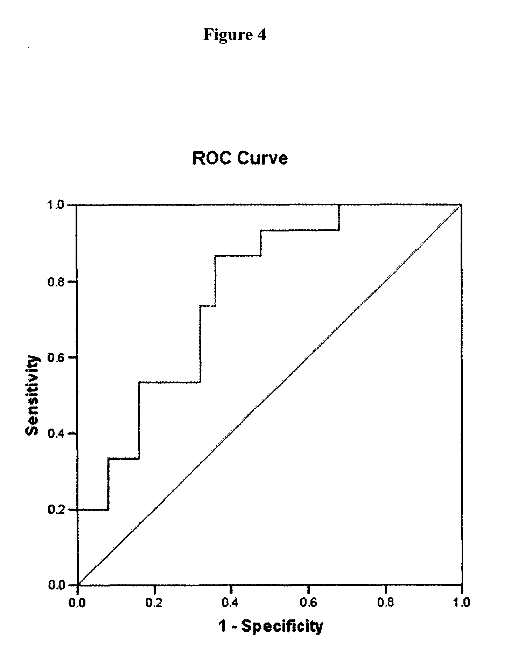

Figure 4 shows a ROC curve illustrating the specificity and sensitivity of one embodiment of the present invention.
Figure 5 shows a ROC curve illustrating the specificity and sensitivity of another embodiment of the present invention.
Figure 6 shows real-time PCR data relating to 3.4kb mtDNA deletion levels associated with breast cancer.
Figure 7 shows a ROC curve illustrating the specificity and sensitivity of another embodiment of the present invention.

### DETAILED DESCRIPTION OF THE INVENTION

As used herein, "cycle threshold" (C_{T}) is the point at which target amplification using real-time PCR rises above background, as indicated by a signal such as a fluorescence signal. The C_{T} is inversely related to the quantity of the sequence being investigated.

As defined herein, "sensitivity" refers to the fraction of true positives (true positive rate) results obtained using the method of the present invention.

As defined herein, "specificity" refers to the fraction of false positives (false positive rate) results obtained using the method of the present invention.

In one embodiment of the present invention, methods are provided for monitoring and diagnosing cancer through the detection and quantification of the aforementioned 3.4 kb mtDNA deletion. For example, the present invention may be used for detecting the presence of pre-neoplasia, neoplasia and progression towards potential malignancy of breast cancer. In one aspect, the present invention involves the detection and quantification of the 3.4kb mtDNA deletion (SEQ ID NO:1) for the detection, diagnosis, and/or monitoring of cancer. In this method, mtDNA is extracted from a biological sample (for example body tissue, or body fluids such as urine, prostate massage fluid). The extracted mtDNA is then tested in order to determine the levels (ie. quantity) of the 3.4 kb deletion in the sample. In tests conducted by the present inventors, the levels of the deletion were found to be elevated in samples obtained from subjects with cancer when compared to samples obtained from subjects without cancer. Based on the information and data supplied below, the inventors have concluded that elevated levels of the 3.4 kb deletion in the mtDNA is indicative of cancer.

As disclosed in PCT WO/06/111029, the 3.4kb deletion spans approximately nucleotides 10744 to 14124 of the mtDNA genome. The mtDNA genome is listed as SEQ ID NO:8 (Genbank accession no. AC_000021). The inventors have determined, as provided by example below, that this deletion is also associated with cancer and in particular prostate and breast cancer. Therefore, such deletion provides an accurate biomarker and, therefore, a valuable tool for the detection, diagnosis, or monitoring of cancer in at least these tissues.

The deletion results in the creation of two deletion monomers, one of 3.4kb in size (small sublimon) and one of approximately 12.6kb in size (large sublimon). The occurrence of the deletion may be detected by either identifying the presence of the small sublimon, or by determining that the 3.4 kb sequence has been deleted from the large sublimon.

As discussed above, the deletion is approximately 3379 bp, and comprises genes encoding NADH dehydrogenase subunit 4L, NADH dehydrogenase subunit 4, NADH dehydrogenase subunit 5, tRNA histidine, tRNAserine2, and tRNA leucine2.

In one embodiment, samples of, for example urine or breast tissue, are obtained from an individual and tested over a period of time (eg. years) in order to monitor the genesis or progression of cancer. Increasing levels of the 3.4 kb deletion over time could be indicative of the beginning or progression of cancer.

Age related accumulation of the 3.4 kb mtDNA deletion may predispose an individual to, for example, prostate cancer or breast cancer, which is prevalent in middle aged and older men, and middle aged and older women, respectively. According to one aspect of the invention, a method is provided wherein regular cancer screening may take place by monitoring over time the amount of the 3.4 kb deletion in body tissues such as breast tissue or body fluids such as urine.

The system and method of the present invention may be used to detect cancer at an early stage, and before any histological abnormalities. For example, the system and method of the present invention may be used to detect pre-neoplasia in breast tissue.

The following primer sequences are preferred for the detection of the 3.4 kb deletion:
3.4 forward (binds to bases 10729-10743/14125-14139 of the mtDNA genome) 5'-TAGACTACGTACATACTAACCCTACTCCTA-3' (SEQ ID NO: 2);
3.4 reverse (binds to bases 14361-14379 of the mtDNA genome) 5'-GAGGTAGGATTGGTGCTGT-3' (SEQ ID NO: 3).

In one embodiment of the present invention, a pair of amplification primers are used to amplify a target region indicative of the presence of the 3.4 kb deletion. In this embodiment, one of the pair of amplification primers overlaps a spliced region of mtDNA after deletion of the 3.4 kb sequence has occurred (ie. a splice at a position between 10743 and 14125 of the mtDNA genome). Therefore, extension of the overlapping primer can only occur if the 3.4 kb section is deleted.

In another embodiment of the present invention, a pair of amplification primers are used to amplify a target region associated with the deleted 3.4 kb sequence. The deleted 3.4 kb sequence, upon deletion, may reform as a circular mtDNA molecule. In this embodiment, one of the pair of amplification primers overlaps the rejoining site of the ends of the 3.4 kb sequence. Thus, an increase in the amount of the 3.4 kb molecule detected in a sample is indicative of cancer. The below primer pair is preferred for the detection of the deleted 3.4 kb nucleic acid.
Forward 14115/10755 5'-CCCACTCATCACCTAAACCTAC-3' (SEQ ID NO: 9)
Reverse 10980R 5'-GGTAGGAGTCAGGTAGTTAG-3' (SEQ ID NO: 10).

In one aspect of the invention, a kit for diagnosing cancer, for example breast cancer, comprising means for extraction of mtDNA, primers having the nucleic acid sequences recited in SEQ ID NOS: 2 and 3, or SEQ ID NOS: 9 and 10, reagents and instructions, is provided.

Another aspect of the invention provides methods for confirming or refuting the presence of a cancer biopsy test from a biopsy sample (eg. breast cancer), comprising: obtaining non-cancerous tissue from a biopsy sample; and detecting and quantifying the amount of the 3.4 kb mtDNA deletion in the non-diseased tissue.

In one embodiment the present invention provides a method for screening individuals for breast cancer from a body fluid sample comprising; obtaining a body fluid sample, and detecting and quantifying the level of the 3.4 kb mtDNA deletion in the body fluid.

Although real-time quantitative PCR methods, as described in the examples below, represent the preferred means for detecting and quantifying the presence or absence of the 3.4kb deletion, other methods that would be well known to an individual of skill in the art could also be utilized. For example quantification of the deletion could be made using Bio-Rad's Bioplex™ System and Suspension Array technology. Generally, the method requires amplification and quantification of sequences using any known methods.

The examples provided below illustrate that this deletion can be used to detect the presence of cancer in biological samples, for example urine, and breast tissue. Based on the findings in these examples, the 3.4 kb mtDNA deletion may be used as a biomarker for cancer.

The various examples provided illustrate a difference in the amount of mtDNA having the 3.4 kb deletion between samples obtained from subjects having cancer, and subjects without cancer. The amount of the 3.4 kb deletion was found to be higher in the samples obtained from subjects having cancer. This determination was made by comparing the amount of the 3.4 kb deletion in the test samples with amounts from known cancer cells and/or known non-cancer cells.

### Example 1: 3.4 kb Deletion in the mtDNA of Prostate Tissue

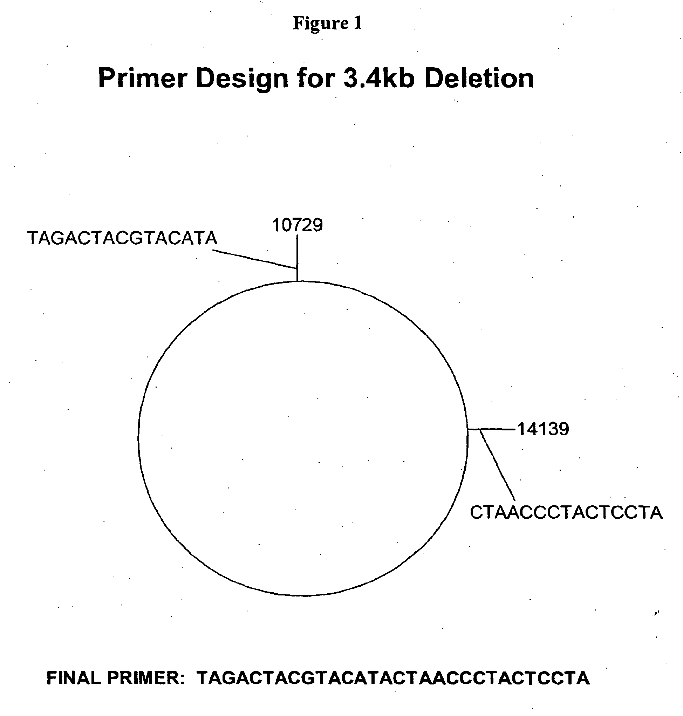

A deletion of approximately 3.4 kilobases (kb) was identified through full mitochondrial genome amplification of fresh frozen prostate tissue. Using linear regression, the size of the deletion was estimated to be between 3000 base pairs (bp) and 3500 bp. Two possible candidate deletions were identified using Mitomap™ (Brandon, M. C., Lott, M. T., Nguyen, K. C., Spolim, S., Navathe, S. B., Baldi, P. & Wallace, D. C., MITOMAP: a human mitochondrial genome database--2004 update. Nucleic Acids Research 33 (Database Issue):D611-613, 2005; www.mitomap.org), the 3397 bp deletion at 9574-12972, and the 3379 bp deletion at 10744-14124. In order to determine which of the two deletions was associated with prostate cancer, if either, a forward primer which bridged the deletion junction was developed for each of the two candidates, ensuring that the primer extended further than the repeat regions that flank the deletions. Figure 1 is a schematic diagram showing the design and sequence of the primer (ie. SEQ ID NO: 2). Positive amplification results for the amplicon corresponding to the 3379 bp deletion (referred to as the 3.4 kb deletion) at 10744-14124 were obtained.

As indicated above, the 3.4 kb deletion removes all or part of the following genes: (i) NADH dehydrogenase subunit 4L, (ii) NADH dehydrogenase subunit 4, (iii) NADH dehydrogenase subunit 5, (iv) tRNA histidine, (v) tRNA serine2, and (vi) tRNA leucine2.

The 3.4kb deletion was determined to be present in 91% of 33 fresh frozen prostate samples. With the specific deletion primers, formalin fixed tissues were tested in order increase the n value.

The present investigators sequenced entire mitochondrial genomes from 32 tissue samples microdissected by laser capture microdisection and 12 needle biopsies from histologically normal prostates. Archived tissue sections from each of these samples were used for the following study. 1-2 serial sections were removed from each sample. DNA was extracted from each sample in its entirety rather than as a microdissection. Thus, each sample consisted of a mixture of glandular prostate tissue as well as stromal prostate tissue. This extraction was performed using Qiagen's QIAamp™ DNA Mini Kit (Cat # 51304). Following extraction the samples were quantified using a Nano-Drop™ spectrophotometer and the concentrations were subsequently normalized to 2ng/ul. Each sample was amplified using 20ng input DNA and an iQ^{™} SYBR Green Supermix™ kit (Bio-Rad Laboratories Inc.) Reactions were run on an Opticon® 2 two colour real-time PCR system (MJ Research).

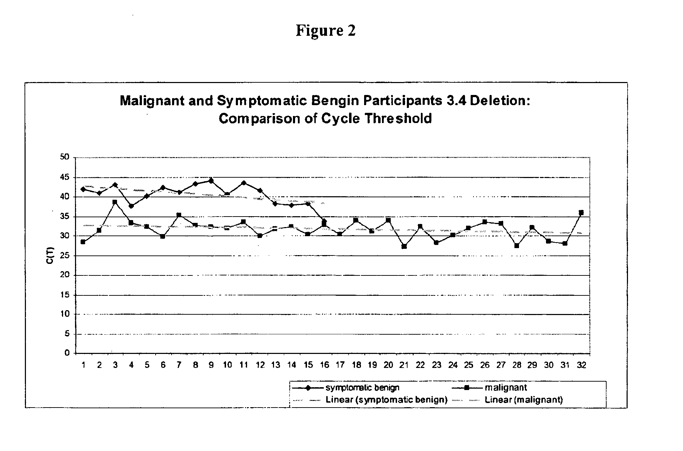

As shown in Figure 2, a distinct difference was observed in cycle threshold and, by extension, quantity of the deletion between the malignant prostate samples and the symptomatic benign prostate samples. Malignant samples exhibited a consistently earlier cycle threshold than the benign samples.

### Example 2: 3.4kb Deletion Blinded Study - Comparison of Cycle Threshold

An additional 21 prostate tissue samples were selected, 10 of which were benign and 11 of which were malignant. The pathological status was determined by needle biopsies conducted by a qualified pathologist. The samples were blinded such that the present investigators were unaware of their pathological status when they conducted this test. The present investigators were able to predict pathological status correctly in 81 % of the cases by examining the cycle threshold. Of the 4 incorrect calls, two were malignant samples that were determined to be benign and 2 were benign samples that were determined to be malignant. Follow-up clinical information for the 2 individuals in the latter scenario was requested from the physician to determine if they had been diagnosed with prostate cancer subsequent to the needle biopsy results used for this study. One of the individuals who originally produced a benign sample but was predicted by this study to have a malignancy subsequently produced a malignant sample. As a result, one of the false positives became a true positive. Therefore, pathological status was predicted correctly in 86% of the cases examined in this study. The ultimate positive predictive value (PPV, where PPV=true positives/(true positives+false positives)) for this study was 91 % and the negative predictive value (NPV, where NPV=true negatives/(true negatives+false negatives)) was 80%.

### Example 3: 3.4kb Deletion Study - Methods (n=76)

Seventy-six prostate tissue samples were examined for the 3.4 kb deletion in this study. All tissue samples were formalin-fixed, 25 being malignant, 12 being normal, and 39 having benign prostatic disease as shown histologically. Of the latter group more then half had hyperplasia. All specimens were needle biopsies taken from the investigators' tissue archives.

### Prostate Specimens

A tapelift was performed on each slide using Prep-Strips (Catalogue Number LCM0207) from Arcturus Bioscience Inc. This allowed the removal of any particulate matter or non-adhering tissue from the slide prior to DNA extraction. With the tissue still on the slides, the slides were rinsed with PBS (Phosphate Buffered Saline Solution) to remove as much fixative as possible. The 1-2 needle biopsy sections on the slides were scraped into sterile microcentrifuge tubes using individually wrapped, sterilized surgical razor blades. DNA was then isolated and purified using a QIAamp^{®} DNA Mini Kit (Qiagen, Cat. # 51304) according to manufacturer's specifications. A negative extract control was processed in parallel with the slide extractions as a quality control checkpoint. The total concentration of DNA and purity ratio for each sample was determined by spectrophotometry (Nano-Drop™ ND-1000) and dilutions of 2ng/µl were prepared for the purpose of Quantitative Polymerase Chain Reaction (qPCR).

### Primers (Oligonucleotides)

Purified oligonucleotide primers were chemically synthesized by Invitrogen (California, USA). The sequences of the primers and the expected sizes of the PCR products amplified are listed in Table 1. In addition, PCR analysis for mtDNA deletions included positive controls (DNA from a source known to carry the mutant mtDNA). Each primer set with the exception of TNF (tumor necrosis factor) were checked against a mitochondria-free rho 0 cell line to confirm the absence of pseudogene coamplification.

**Table 1 Amplification Primers.**

| **Primer Pair** | **Position Amplified 5'- 3'** | **Length of amplified product (base pairs)** |
|---|---|---|
| **3.4 Deletion Real-Time** | 10729-14379 (less 3379bp at 10744-14124) | 273 |
| **12s mtDNA** | 708-945 | 238 |
| **TNF** | 3756-3886 | 131 |
| **3.4** | forward (10729-10743 - 14125-14139) 5'TAGACTACGTACATACTAACCCTACTCCTA-3' SEQ ID NO: 2 | |
| **3.4** | reverse (14361-14379) 5'-GAGGTAGGATTGGTGCTGT-3' SEQ ID NO: 3 | |
| **12s** | forward (708-728) 5'-CGTTCCAGTGAGTTCACCCTC-3" SEQ ID NO: 4 | |
| **12s** | reverse (923-945) 5'-CACTCTTTACGCCGGCTTCTATT-3' SEQ ID NO: 5 | |
| **TNF** | forward (3756-3775) 5' -CCTGCCCCAATCCCTTTATT-3' SEQ ID NO: 6 | |
| **TNF** | reverse (3866-3886) 5'-GGTTTCGAAGTGGTGGTCTTG-3'SEQ ID NO: 7 | |

### Real-Time Polymerase Chain Reaction

Three separate PCRs were performed on each sample. Each reaction was 25µl total volume and included template DNA, one pair of primers (12s or 3.4 Deletion or TNF), an iQ^{™} SYBR Green Supermix™ kit (Catalogue Number 170-8882, Bio-Rad Laboratories Inc.) and distilled deionized water (ddH₂O). The TNF (tumor necrosis factor) comprised single copy nuclear gene primers, and 12s comprised total mitochondrial genome primers. The volume and concentrations for template DNA, primers, and reaction buffer are listed below.

**Table 2 qPCR Components.**

| **Reagent** | **Concentration per Reaction** | **Volume per Reaction** |
|---|---|---|
| Reaction Buffer | 1X | 12.5µl |
| Primer (forward and reverse) | 250nM | 0.0625µl of each 100 umole stock |
| ddH₂O | N/A | 2.375.µl |
| Template DNA | 20ng | 10.0µl |
| **Total** | | 25µl |

The cycling parameters for each amplicon are listed in Table 3.

**Table 3 Cycling Parameters.**

| Step | Temperature (°C) | Duration |
|---|---|---|
| 1 | 95 | 3 min |
| 2 | 95 | 30 sec |
| 3 | 66 (3.4 deletion primers) or | 30 sec |
| | 61.5 (12s primers) or | |
| | 61.5 (TNF primers) | |
| 4 | 72 | 30 sec |
| 5 | Plate Read | |
| 6 | 72 | 10 min |
| 7 | Melting Curve 50°C - 110°C reading every 1 °C | 3 sec |
| Repeat steps 2-5, 44 times for a total of 45 cycles. | | |

Thermal cycling, real-time detection and analysis of the reactions was carried out using a DNA Engine Opticon^{®} 2 Continuous Fluorescence Detection System equipped with Intuitive Opticon Monitor^{™} software (MJ Research Inc.). The standard curve method was utilized for DNA quantification. A set of serial dilutions (10⁶, 10⁵, 10⁴, 10³, 10², 10¹) of three purified PCR generated templates, one product for the 3.4 deletion, one for the 12s primers, and one for TNF. From this, three different standard curves were generated showing the number of copies of total mtDNA (12s amplicon-total mitochondrial genome primers), the amount of mtDNA having the 3.4 kb deletion, or total nuclear DNA (TNF-single copy nuclear gene primers). The C_{T} values of the samples were then converted to the number of DNA copies by comparing the sample C_{T} to that of the standards. The 3.4 deletion was considered to be absent or at low levels if the deletion was not detected within 37 cycles.

The determination of malignancy is based upon the quantity of the 3.4kb deletion present in the normalized sample as indicated by the location of the cycle threshold. This location may be either absolute, as in greater than 25 cycles but less than 35 cycles, or more likely a ratio between the total mitochondrial DNA present as indicated by the 12s amplicon, and the 3.4kb deletion. This may be expressed as a per cent of the total mitochondrial DNA. The number of cells, as represented by the TNF amplicon, may be incorporated to refine the distinction between benign and malignant tissues.

In order to automate the analyses of these samples, bioinformatics tools were employed. The three variables that were considered for these analyses were the cycle threshold C_{T} of Tumour Necrosis Factor (TNF), total pecies of mitochondria that contain those specific primer sites, and those mitochondria that harbour the deletion of interest.

### Cluster Analysis

The clustering was not normalized nor were logarithmic functions used due to the similar and small range of data.

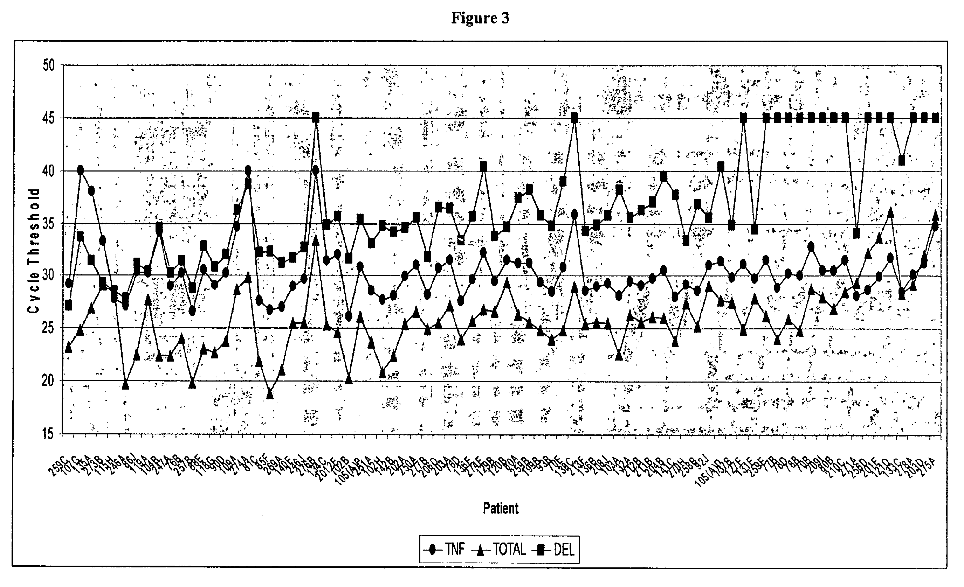

Figure 3 shows the actual movement and trends of the data. The x-axis is the patient number and the y-axis is the cycle threshold obtained from real time PCR.

It is important to note that the higher the cycle threshold is, the lower amount of the deletion is present.

The general trend shown in Figure 3 is based upon the differences/ratios between the variables of Deletion, Total, and TNF. The deletion is low to absent for the benign/normal samples (right side) and increases (toward the left) with abnormal benign and malignant samples. The abnormal benign and malignant samples begin to differentiate themselves from each other based on the cycle threshold ratio of Deletion to TNF.

### Supervised Learning

Supervised learning is based on the system trying to predict outcomes for known samples. Half of the data was used to train and the other half to test the algorithm. Supervised learning compares its predictions to the target answer and "learns" from its mistakes. But, if the predicted output is higher or lower than the actual outcome in the data, the error is propagated back through the system and the weights are adjusted accordingly.

| | |
|---|---|
| Data SET: | 5% to 35% - **Benign** |
| | 35% to 65% - **Hyperplasia** |
| | 65% to 95% - **Malignant** |

Artificial Neural Network (ANN) Algorithm (shown schematically below):
Half of Data set used for Training ANN
Other half used to compare the accuracyAccuracy = Compare expected data set with obtained data set → 86.6%

Supervised Learning of Deletion Data using Artificial Neural Network (ANN)

Three Classifications:
Benign
Hyperplasia
Malignant

Three variables for each classification were used based on Real Time PCR Cycle Threshold C_{T}:
Tumour Necrosis Factor (TNF) - Nuclear copy control.
Total Mitochondria - Mitochondria copy control
Deletion - Mitochondria in the deleted state.

### Results:

Half of data set is used to train the ANN, and the remaining half is used to compare the accuracy.
**Three Classification Accuracy** = 86.6%
**Positive Predictive Value (PPV);**
Benign to Malignant = 88.2%
**Negative Predictive Value (NPV)**
Benign to Malignant = 76.5%

### Example 4: 3.4 kb Deletion in mtDNA Associated with Breast Cancer

18 samples were tested from malignant and benign breast tissue, 9 being malignant and 9 being benign, for the presence of the aforementioned 3.4 kb deletion. Samples were classified as either malignant or benign using conventional histopathological analysis.

DNA was isolated and purified from the samples using a QIAamp^{®} DNA Mini Kit (Qiagen, Cat. # 51304) according to manufacturer's specifications.

Purified oligonucleotide primers were chemically synthesized by Invitrogen (California, USA). The sequences of the primers and the expected sizes of the PCR products amplified are listed in Table 1 above.

### Real-Time Polymerase Chain Reaction

Three separate PCRs were performed on each sample. Each reaction was 25µl total volume and included template DNA, one pair of primers (12s or 3.4 Deletion or TNF), an iQ^{™} SYBR Green Supermix kit (Catalogue Number 170-8882, Bio-Rad Laboratories Inc.) and distilled deionized water (ddH₂O). The TNF (tumor necrosis factor) comprised single copy nuclear gene primers, and 12s comprised total mitochondrial genome primers. The volume and concentrations for template DNA, primers, and reaction buffer are listed below:

**Table 4 qPCR Components.**

| **Reagent** | **Concentration per Reaction** | **Volume per Reaction** |
|---|---|---|
| Reaction Buffer | 1X | 12.5µl |
| Primer (forward and reverse) | 250nM | 0.0625µl of each |
| | | 100 µmole stock |
| ddH₂O | N/A | 2.375.µl |
| Template DNA | 20ng | 10.0µl |
| **Total** | | 25µl |

The cycling parameters for each amplicon are listed in Table 5.

**Table 5 Cycling Parameters.**

| Step | Temperature (°C) | Duration |
|---|---|---|
| 1 | 95 | 3 min |
| 2 | 95 | 30 sec |
| 3 | 66 (3.4 deletion primers) or | 30 sec |
| | 61.5 (12s primers) or | |
| | 61.5 (TNF primers) | |
| 4 | 72 | 30 sec |
| 5 | Plate Read | |
| 6 | 72 | 10 min |
| 7 | Melting Curve 50°C - 110°C reading every 1°C | 3 sec |
| Repeat steps 2-5, 44 times for a total of 45 cycles. | | |

Thermal cycling, real-time detection and analysis of the reactions was carried out using a DNA Engine Opticon^{®} 2 Continuous Fluorescence Detection System equipped with Intuitive Opticon Monitor^{™} software (MJ Research Inc.). The standard curve method was utilized for DNA quantification. A set of serial dilutions (10⁶, 10⁵, 10⁴, 10³, 10², 10¹) of three purified PCR generated templates were performed, one product for the 3.4 deletion, one for the 12s primers, and one for TNF. From this, three different standard curves were generated showing the number of copies of total mtDNA (12s amplicon-total mitochondrial genome primers), 3.4 deletion or total nuclear DNA (TNF-single copy nuclear gene primers). The C_{T} values of the samples were then converted to the number of DNA copies by comparing the sample C_{T} to that of the standards.

The determination of malignancy was based upon the quantity of the 3.4kb deletion present in the normalized sample as indicated by the location of the cycle threshold. This location may be either absolute, as in greater than 25 cycles but less than 30 cycles, or more likely a ratio between the total mitochondrial DNA present as indicated by the 12s amplicon, and the 3.4kb deletion. This may be expressed as a percent of the total mitochondrial DNA.

In order to automate the analyses of these samples, bioinformatics tools were employed. The three variables that were considered for these analyses were the cycle threshold C_{T} of Tumour Necrosis Factor (TNF), total species of mitochondria that contain those specific primer sites, and those mitochondria that harbour the deletion of interest.

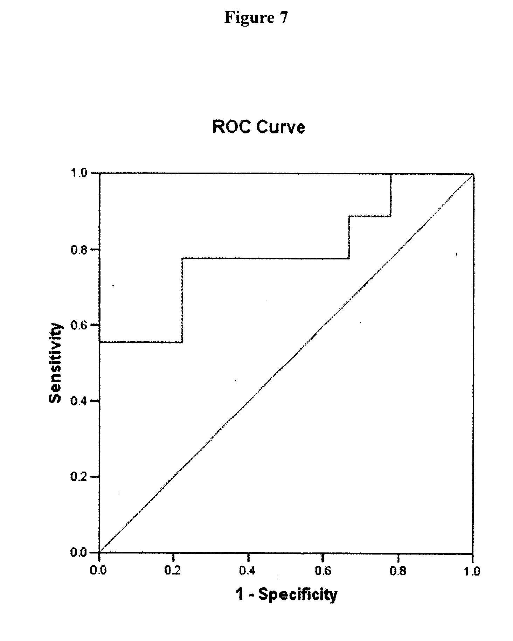

Table 6 and figure 7 show the difference in the mean C_{T} scores for samples from malignant tissue and benign tissue. The mean C_{T} value for normal tissue was 30.5889, while the mean C_{T} for malignant tissue was 27.8533 thereby illustrating a difference in the quantity of mtDNA having the 3.4 kb deletion in malignant breast tissue compared to normal breast tissue.

**Table 6 Mean values for C_{T} scores**

| **Group Statistics** | | | | | |
|---|---|---|---|---|---|
| | GRP | N | Mean | Std. Deviation | Std. Error Mean |
| del3.4 | normal | 9 | 30.5889 | 2.53897 | .84632 |
| | malignant | 9 | 27.8533 | 2.52253 | .84084 |

Figure 8 is an ROC curve illustrating the specificity and sensitivity of the 3.4 kb mtDNA deletion as a marker for breast cancer when testing breast tissue. These results were obtained using a cutoff C_{T} of 29.1900. The sensitivity of the marker at this C_{T} was 77.8%, while the specificity was 77.8%.

Table 7 shows the calculation of the area under the curve for the present example. As a measure of the accuracy of the test.

**Table 7 Results Showing Area Under the Curve**

| **Area Under the Curve** | | | | |
|---|---|---|---|---|
| Test Result Variable(s): del3.4 | | | | |
| Area | Std. Error^{a} | Asymptotic Sig.^{b} | Asymptotic 95% Confidence Interval | |
| | | | Lower Bound | Upper Bound |
| .790 | .112 | .038 | .570 | 1.010 |

| | | | | |
|---|---|---|---|---|
| ^{a.} Under the nonparametric assumption ^{b.} Null hypothesis: true area = 0.5 | | | | |

The determination of the cutoff C_{T} of 29.1900 is shown in table 8 below. The results listed in table 8 show that a cutoff C_{T} of 29.1900 provided the highest sensitivity and specificity at 78% and 78% respectively.

**Table 8: Determination of C_{T}cutoff.**

| **Coordinates or the Curve** | | |
|---|---|---|
| **Test Result Variabla(s): del3.4** | | |
| **Positive if Less Than or Equal To^{a}** | **Senstivity** | **1 - Specificity** |
| 24.6000 | .000 | .000 |
| 25.6800 | .111 | .000 |
| 25.7700 | .222 | .000 |
| 25.9250 | .333 | .000 |
| 26.2050 | .444 | .000 |
| 26.8400 | .556 | .000 |
| 27.4800 | .556 | .111 |
| 28.1600 | .556 | .222 |
| 28.8800 | .667 | .222 |
| 29.1900 | .778 | .222 |
| 29.4600 | .778 | .333 |
| 29.8750 | .778 | .444 |
| 30.5850 | .778 | .556 |
| 31.2200 | .778 | .667 |
| 31.5000 | .889 | .667 |
| 31.7650 | .889 | .778 |
| 32.9900 | 1.000 | .778 |
| 34.3350 | 1.000 | .889 |
| 35.6400 | 1.000 | 1.000 |

| | | |
|---|---|---|
| ^{a.} The smallest cutoff value is the minimum observed test value minus 1, and the largest cutoff value is the maximum observed test value plus 1. All the other cutoff values are the averages of two consecutive ordered observed test values. | | |

### Example 5: The 3.4kb Deletion in the Prostate Massage Fluid of Individuals with Prostate Cancer as Compared to the Fluid from those without Histological Evidence of Prostate Cancer

Forty prostate massage fluid samples were collected by urologists from patients who were either subsequently diagnosed with prostate cancer or showed no histological evidence of prostate cancer following a prostate needle biopsy procedure. The sample was deposited on a IsoCode Card™ (Schleicher & Shuell), dried, and then extracted according to the manufacturer's protocol. All DNA extracts were quantified using a NanoDrop™ ND-1000 Spectrophotometer and the DNA concentration normalized to 2ng/ul. Each sample was then amplified according to the following parameters:
1X iQ SYBR Green Supermix™ (Bio-Rad P/N 170-8880)
150nmol forward primer
(5'-TAGACTACGTACATACTAACCCTACTCCTA-3') (SEQ ID NO: 2).
150 nmol reverse primer
(5'-GAGGTAGGATTGGTGCTGT-3') (SEQ ID NO: 3)
20 ng template DNA
in a 25ul reaction.

Reactions were cycled on an Opticon™ 2 DNA Engine (Bio-Rad Canada) according to the following protocol:
1. 95°C for 3 minutes
2. 95°C for 30 seconds
3. 66°C for 30 seconds
4. 72°C for 30 seconds
5. Plate Read
6. Repeat steps 2-5 44 times
7. 72°C for 10 minutes
8. Melting Curve from 50°C to 105°C, read every 1°C, hold for 3 seconds
9. 10°C Hold

**Table 9 Results showing the mean C_{T} Values for Prostate Massage Fluid Test**

| Group Statistics | | | | | |
|---|---|---|---|---|---|
| | Group | N | Mean | Std. Deviation | Std. Error Mean |
| DEL3.4 | benign | 25 | 37.1869 | 3.18495 | .63699 |
| | malignant | 15 | 33.7712 | 3.98056 | 1.02778 |

Tables 9 and 10 show a significant difference between the mean C_{T} values obtained for the benign sample and the malignant sample groups (p=0.005).

**Table 10 Results Showing Difference (p=0.005) for C_{T} values of samples.**

| **Independent Samples Test** | | | | | | | | | | |
|---|---|---|---|---|---|---|---|---|---|---|
| | | Levene's Test for Equality of Variances | | t-test for Equality of Means | | | | | | |
| | | | | | | | | | 95% Confidence Interval of the Difference | |
| | | F | Sig. | t | df | Sig. (2-tailed) | Mean Difference | Std. Error Difference | Lower | Upper |
| DEL3.4 | Equal variances assumed | 1.251 | .270 | 2989 | 38 | .005 | 3.41570 | 1.14283 | 1.10217 | 5.72923 |
| | Equal variances not assumed | | | 2825 | 24.696 | .009 | 3.41570 | 1.20917 | .92382 | 5.90758 |

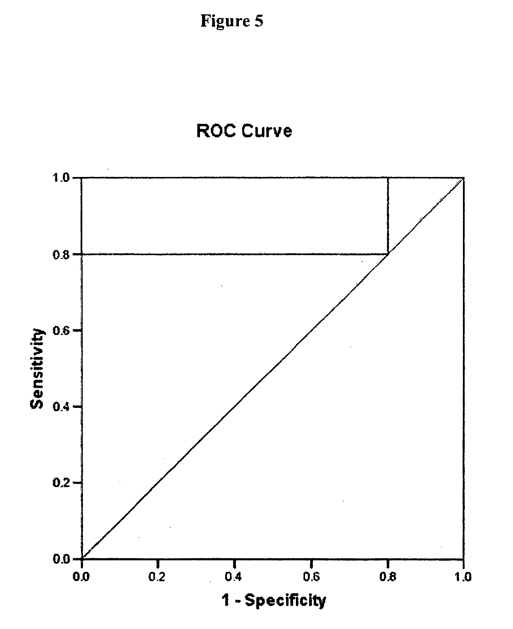

Figure 5 is a Receiver Operating Characteristic (ROC) curve illustrating the specificity and sensitivity of the 3.4 kb mtDNA deletion as a marker for prostate cancer when testing prostate massage fluid. These results were obtained using a cutoff C_{T} of 37.3683. The sensitivity of the marker at this C_{T} is 87%, while the specificity is 64%.

The accuracy of the test depends on how well the test separates the group being tested into those with and without the prostate cancer. Accuracy is measured by the area under the ROC curve. Table 11 shows the calculation of the area under the curve for the present example.

**Table 11 Results Showing Area Under the ROC Curve**

| **Area Under the Curve** | | | | |
|---|---|---|---|---|
| Test Result Variable(s): DEL 3.4 | | | | |
| Area | Std. Error^{a} | Asymptotic Sig.^{b} | Asymptotic 95% Confidence Interval | |
| | | | Lower Bound | Upper Bound |
| .768 | .074 | .005 | .622 | .914 |

| | | | | |
|---|---|---|---|---|
| ^{a.} Under the nonparametric assumption ^{b.} Null hypothesis: true area = 0.5 | | | | |

**Table 12 Determination of Specificity and Sensitivity**

| **Coordinates of the Curve** | | |
|---|---|---|
| Test Result Variable (s): DEL3.4 | | |
| Positive if Less Than or Equal To ^{a} | Senstivity | 1 -Specificity |
| 26.2992 | .000 | .000 |
| 27.3786 | .067 | .000 |
| 28.2484 | .133 | .000 |
| 29.5193 | .200 | .000 |
| 30.1757 | .200 | .040 |
| 30.4580 | .200 | .080 |
| 30.5980 | .267 | .080 |
| 31.5709 | .333 | .080 |
| 32.5712 | .333 | .120 |
| 32.9500 | .333 | .160 |
| 33.3314 | .400 | .160 |
| 33.6547 | .467 | .160 |
| 33.9247 | .533 | .160 |
| 34.3554 | .633 | .200 |
| 34.9056 | .533 | .240 |
| 35.4650 | .533 | .280 |
| 35.9172 | .533 | .320 |
| 36.0648 | .600 | .320 |
| 36.3616 | .667 | .320 |
| 36.6421 | .733 | .320 |
| 36.8531 | .733 | .360 |
| 37.1188 | .800 | .360 |
| 37.3683 | .867 | .380 |
| 37.5200 | .867 | .400 |
| 37.8341 | .867 | .440 |
| 38.2533 | .867 | .480 |
| 38.5198 | .933 | .480 |
| 38.6519 | .933 | .520 |
| 38.8552 | .933 | .580 |
| 39.1258 | .933 | .600 |
| 39.2734 | .933 | .640 |
| 39.4952 | .933 | .680 |
| 39.7323 | 1.000 | .680 |
| 39.8956 | 1.000 | .720 |
| 41.0000 | 1.000 | 1.000 |

The smallest cutoff value is the minimum observed test value -1, and the largest cutoff value is the maximum observed test value plus 1. All the other cutoff values are the average of two consecutive ordered, observed test values.

The determination of the cutoff C_{T} of 37.3683 is shown in table 12 above. The results listed in table 12 illustrate that a cutoff C_{T} of 37.3683 provided the highest sensitivity and specificity.

### Example 6: The 3.4kb Deletion in the Urine of Individuals with Prostate Cancer as Compared to the fluid from those without Histological Evidence of Prostate Cancer

Urine samples were collected from 5 patients who were diagnosed with prostate cancer and 5 who have had a needle biopsy procedure which was unable to detect prostate malignancy. These samples were collected following a digital rectal exam (DRE) to facilitate the collection of prostate cells.

Upon receipt of the samples a 5ml aliquot was removed and then 2mls were centrifuged at 14,000 x g to form a pellet. The supernatant was removed and discarded. Pellets were resuspended in 200ul phosphate buffered saline solution. Both the resuspended pellet and the whole urine sample were subjected to a DNA extraction procedure using the QiaAMP™ DNA Mini Kit (Qiagen P/N 51304) according to the manufacturer's directions. The resulting DNA extracts were then quantified using a NanoDrop™ ND-1000 Spectrophotometer and normalized to a concentration of 0.1ng/ul.

Samples were analyzed by quantitative real-time PCR with the 3.4kb deletion specific primers according to the following:
1X iQ SYBR Green Supermix™ (Bio-Rad P/N 170-8880)
100 nmol forward primer (5'-TAGACTACGTACATACTAACCCTACTCCTA-3') (SEQ ID NO: 2)
100 nmol reverse primer (5'-GAGGTAGGATTGGTGCTGT-3') (SEQ ID NO: 3)
1 ng template DNA
in a 25ul reaction.

Reactions were cycled on an Opticon™ 2 DNA Engine (Bio-Rad Canada) according to the following protocol:
1.95°C for 3 minutes
2. 95°C for 30 seconds
3. 69°C for 30 seconds
4. 72°C for 30 seconds
5. Plate Read
6. Repeat steps 2-5 44 times
7. 72°C for 10 minutes
8. Melting Curve from 50°C to 105°C, read every 1°C, hold for 3 seconds
9. 10°C Hold

**Table 13 Mean values for C_{T} scores**

| Group Statistics | | | | | |
|---|---|---|---|---|---|
| | GRPfluid38 | N | Mean | Std. Deviation | Std. Error Mean |
| CTf3.4 | Benign | 5 | 33.2780 | 1.10900 | .49596 |
| | Malignant | 5 | 30.6980 | 2.55767 | 1.14382 |

Tables 13 and 14 show a significant difference between the mean C_{T} values obtained for benign sample and the malignant sample groups (p=0.005).

**Table 14 Results Showing Difference (p=0. 005) for C_{T} values of samples.**

| **Independent Samples Test** | | | | | | | | | | |
|---|---|---|---|---|---|---|---|---|---|---|
| | | Levene's Test for Equality of Variances | | t-test for Equality of Means | | | | | | |
| | | | | | | | | | 95% Confidence Interval of the Difference | |
| | | F | Sig. | t | df | Sig. (2-tailed) | Mean Difference | Std. Error Difference | Lower | Upper |
| CTf | Equal variances assumed | 1.272 | .292 | 2069 | 8 | .072 | 258000 | 1.24672 | -.29494 | 5.45494 |
| | Equal variances not assumed | | | 2069 | 5.453 | .089 | 258000 | 1.24672 | -.54639 | 5.70639 |

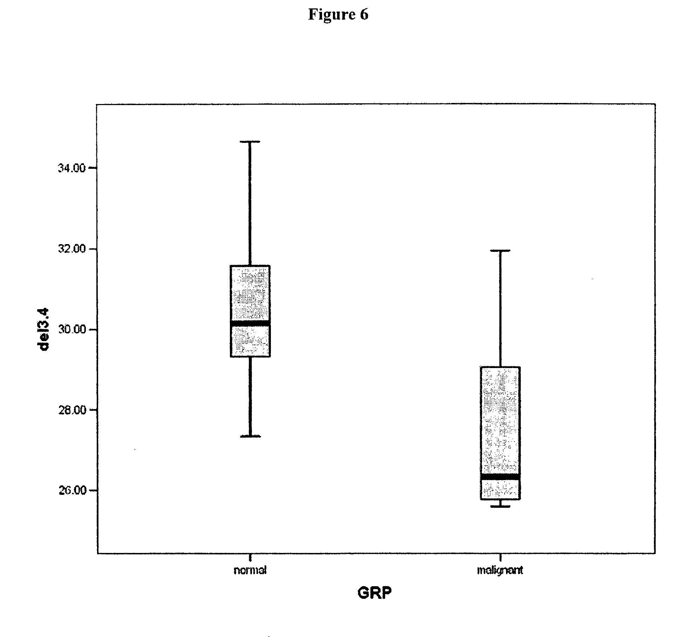

Figure 6 is a Receiver Operating Characteristic (ROC) curve illustrating the specificity and sensitivity of the 3.4 kb mtDNA deletion as a marker for prostate cancer when testing urine. These results were obtained using a cutoff C_{T} of 31.575. The sensitivity of the marker at this C_{T} is 80%, while the specificity is 100%.

The determination of the cutoff C_{T} of 31.575 is shown in table 15. The results listed in table 15 show that a cutoff C_{T} of 31.575 provided the highest sensitivity and specificity.

**Table 15: Determination of C_{T} cutoff.**

| **Coordinates of the Curve** | | |
|---|---|---|
| Test Result Variable(s); CTf | | |
| Positive if LessThan nr Equal To^{a} | Sensitivity | 1 - Specificity |
| 26.2900 | .000 | .000 |
| 28.4950 | .200 | .000 |
| 30.3850 | .400 | .000 |
| 31.0800 | .600 | .000 |
| 31.5750 | .800 | .000 |
| 32.1400 | .800 | .200 |
| 32.8150 | .800 | .400 |
| 33.8700 | .800 | .600 |
| 34.3350 | .800 | .800 |
| 34.3550 | 1.000 | .800 |
| 35.3700 | 1.000 | 1.000 |

| | | |
|---|---|---|
| ^{a.} The smallest cutoff value is the minimum observed test value minus 1, and the largest cutoff value is the maximum observed test value plus 1. All the other cutoff values are the averages of two consecutive ordered observed test values. | | |

### Example 7: Detection of Re-circularized 3.4kb Deleted Sequence in Prostate Malignant and Benign Tissue

In this example, the amount of re-circularized 3.4 kb deleted mtDNA molecules in samples was tested as an indicator for prostate cancer. As mentioned above, the 3.4 kb sequence, upon deletion, may reform as a circular mtDNA molecule. Amplification of a target region from the deleted 3.4 kb mtDNA sublimon was conducted using a primer pair (SEQ ID NOS: 9 and 10). The forward primer (SEQ ID NO: 9), overlaps the rejoining site of the ends of the 3.4 kb sequence.

Prostate tissue was formalin-fixed paraffin embedded prostate tissue needle biopsies.

The reagent setup used for this example was as follows:
250nmol each primer
12.5ul of 2X reaction mix,
20ng (10ul of 2ng/ul) template in 25 ul reaction volume.

The cycling parameters were as follows:
1. 95 degrees Celsius for 3 minutes
2. 95 degrees Celsius for 30 seconds
3. 62 degrees Celsius for 30 seconds
4. 72 degrees Celsius for 30 seconds
5. Plate Read
6. Repeat steps 2-5 44 times
7. 72 degrees for 10 minutes
8. Melting Curve from 50-100 degrees, reading every 1 degree for 3 seconds
9. 4 degrees HOLD.

Amplification of a target region from the deleted 3.4 kb mtDNA sublimon was conducted using a primer pair (SEQ ID NOS: 9 and 10).

Table 16 below provides a summary of testing conducted for the detection of the actual 3.4 kb deleted in mtDNA obtained from malignant and benign prostate tissue. Using a C_{T} score of 30.0, a clear identification of malignant and benign tissue was possible. As such, an increase in the amount of the 3.4 kb molecule present in a sample was indicative of cancer.

**Table 16: C_{T} scores for Detection of Cancer in Prostate Tissue**

| **Description** | **C_{T}** |
|---|---|
| Benign sample 1 | 33.75 |
| Malignant sample 1 | 28.79 |
| Benign sample 2 | 30.96 |
| Malignant sample 2 | 28.4 |
| Benign sample 3 | 32.19 |
| Malignant sample 3 | 27.38 |

### REFERENCES

Birch-Machin MA, Online Conference Report (Sunburnt DNA), International Congress of Biochemistry and Molecular Biology, New Scientist, 2000(a)
Birch-Machin MA, Taylor RW, Cochran B, Ackrell BAC, Tumbull DM. Ann Neurol48: 330-335, 2000(b)
Birch-Machin, M.A. (2000). Mitochondria and skin disease. Clin Exp Dermatol, 25, 141-6.
Brown, M.D., et al., Am J. Humn Genet, 60: 381-387, 1997
Bogliolo, M, et al., Mutagenesis, 14: 77-82, 1999
Chinnery PF and Turnbull DM., Lancet 354 (supplement 1): 17-21, 1999
Huoponen, Kirsi, Leber hereditary optic neuropathy: clinical and molecular genetic findings, Neurogenetics (2001) 3: 119-125.
Hayward SW, Grossfeld GD, Tlsty TD, Cunha GR., Int J Oncol 13:35-47, 1998
Huang GM, Ng WL, Farkas J, He L, Liang HA, Gordon D, Hood R., Genomics 59(2):178-86,1999
Konishi N, Cho M, Yamamoto K, Hiasa Y. Pathol. Int. 47:735-747,1997
Landis SH, Murray T, Bolden S, Wingo PA. Cancer J. Clin. 49:8-31
Lee HC, Lu CY, Fahn HJ, Wei YHu. Federation of European Biochemical Societies, 441:292-296,1998
Mitochondrial Research Society http://www.mitoresearch.org/diseases.html.
MITOMAP: A human mt genome database (www.sen.emory.edu/mitomap.html)
Naviaux, RK., Mitochondrial Disease- Primary Care Physican's Guide. Psy-Ed. Corp D/B/A Exceptional Parents Guide: 3-10, 1997
Parrella P, Xiao Y, Fliss M, Sanchez-Cespedes M, Mazzarelli P, Rinaldi M, Nicol T, Gabrielson E, Cuomo C, Cohen D, Pandit S, Spencer M, Rabitti C, Fazio VM, Sidransky D: Detection of mitochondrial DNA mutations in primary breast cancer and fine-needle aspirates. Cancer Res 2001, 61:7623-7626
Polyak Y, et al., Nature Genet. 20 (3):291-293, 1998
Seidman, M.D. et al., Arch. Otolaryngol Head Neck Surg., 123: 1039-1045, 1997
Sherrat EJ, Thomas AW, Alcolado JC., Clin. Sci. 92:225-235,1997
Shoffner JM, Brown MD, Torroni A, Lott MT, Cabell MF, Mirra SS, Beal MF, Yang C, Gearing M, Salvo R, Watts RL, Juncos JL, Hansen LA, Crain BJ, Fayad M, Reckford CL, and Wallace DC., Genomics 17: 171-184, 993
SpringNet - CE Connection: Screening, Diagnosis: Improving Primary Care Outcomes. Website: http://www.springnet.com/ce/j803a.htm
Taniike, M. et al., BioChem BioPhys Res Comun, 186: 47-53, 1992
Valnot, Isabelle, et al., A mitochondrial cytochrome b mutation but no mutations of nuclearly encoded subunits in ubiquinol cytochrome c reductase (complex III) deficiency, Human Genetics (1999) 104: 460-466
von Wurmb, N, Oehmichen, M, Meissner, C., Mutat Res. 422:247-254, 1998
Wallace et al., Mitochondiral DNA MUtatio Assoicated with Leber's Hereditary Optic Neuropathy, Science, 1427-1429
Wei YH. Proceedings of the Nat. Sci. Council of the Republic of China April 22(2):5567, 1998
Woodwell DA. National Ambulatory Medical Care Survey: 1997 Summary. Advance data from vital and health statistics; no. 305. Hyattsville, Maryland: National Center for Health Statistics. 1999
Tan Duan-Jun et al., Cancer Research, 62(4): 972-6, 2002
Yeh, J.J., et al., Oncogene Journal, 19: 2060-2066, 2000
Zhang et al., Multiple mitochondiral DNA deletions in an elderly human individual, FEBS Lett, 297,34-38 1992
Zhang, C., et al., BioChem. BioPhys. Res. Comun., 195: 1104-1110, 1993
Zhu et al., Cancer detection and prevention, 28(2):119-26, 2004

## Claims

1. A method of detecting a cancer in an individual comprising:
a) extracting mitochondrial DNA, mtDNA, from a biological sample from the individual;
b) quantifying the amount of mtDNA in the sample having a deletion of approximately 3379 base pairs in the nucleic acid sequence spanning approximately residues 10744 to 14124 of the mtDNA genome;
c) comparing the amount of mtDNA in the sample having the deletion to at least one known reference value;
wherein the cancer is breast cancer.

2. The method of claim 1, wherein the at least one known reference value is the amount of the deletion in a reference sample of mtDNA from known non-cancerous tissue or body fluid.

3. The method of claim 1, wherein the at least one known reference value is the amount of the deletion in a reference sample of mtDNA from known cancerous tissue or body fluid.

4. The method of claim 1, wherein the known reference value is the amount of the deletion in a reference sample of mtDNA from known non-cancerous tissue or body fluid,
wherein an elevated amount of the deletion in the biological sample compared to the reference sample is indicative of cancer.

5. The method of claim 4, further comprising the step of comparing the amount of mtDNA in the sample having the deletion to the amount of the deletion in a reference sample of mtDNA from known cancerous tissue or body fluid.

6. The method of claim 1, wherein the known reference value is the amount of the deletion in a reference sample of mtDNA from known cancerous tissue or body fluid,
wherein a similar level of the deletion in the biological sample compared to the reference sample is indicative of cancer.

7. The method of claim 6, further comprising the step of comparing the amount of mtDNA in the sample having the deletion to the amount of the deletion in a reference sample of mtDNA from known non-cancerous tissue or body fluid.

8. A method of monitoring an individual for the development of a cancer comprising:
a) extracting mtDNA from a biological sample from the individual;
b) quantifying the amount of mtDNA in the sample having a deletion of approximately 3379 base pairs in the nucleic acid sequence spanning approximately residues 10744 to 14124 of the mtDNA genome;
c) repeating steps a) to b) over a duration of time,
wherein an increasing level of the deletion over the duration of time is indicative of cancer;
wherein the cancer is breast cancer.

9. The method of claim 8, further comprising at least one step selected from the group consisting of: (a) comparing the amount of mtDNA in the sample having the deletion to the amount of the deletion in a reference sample of mtDNA from known non-cancerous tissue or body fluid; and (b) comparing the amount of mtDNA in the sample having the deletion to the amount of the deletion in a reference sample of mtDNA from known cancerous tissue or body fluid.

10. The method of any one of the preceding claims, wherein the deletion has a nucleic acid sequence corresponding to the sequence identified in SEQ ID NO: 1.

11. The method of any one of the preceding claims, wherein the quantifying of the deletion includes first amplifying a target region of mtDNA that is indicative of the deletion, and quantifying the amount of the amplified target region.

12. The method according to claim 11, wherein the amplifying of the target region is conducted using a pair of amplification primers, one of the pair of amplification primers overlapping a splice joining regions on opposite ends of the deletion.

13. The method of any one of the preceding claims, wherein the step of quantifying is conducted using real-time PCR.

14. The method of claim 1, wherein the step of quantifying is conducted using real-time PCR and wherein the reference value is a cycle threshold.

15. The method of claim 11 or 14, wherein a PCR primer having a sequence corresponding to SEQ ID NO: 2 is used as part of a pair of amplification primers for amplifying the target region.

16. The method of claim 11 or 14, wherein one of a pair of PCR primers used in the amplifying of the target region overlaps a spliced region of mtDNA after deletion of the sequence corresponding to SEQ ID NO: 1.

17. The method of any one of the preceding claims, wherein the biological sample is a body tissue or body fluid.

18. The method of claim 17, wherein the biological sample is selected from breast tissue and urine.

## Patentansprüche

1. Verfahren zum Nachweis einer Krebserkrankung in einer Person, Schritte umfassend, bei denen man:
a) mitochondriale DNA, mtDNA, aus einer biologischen Probe, die aus der Person stammt, extrahiert;
b) die Menge an mtDNA in der Probe quantifiziert, die eine Deletion von ungefähr 3379 Basenpaaren in der Nukleinsäuresequenz, die ungefähr die Positionen 10744 bis 14124 des mtDNA-Genoms umfasst, aufweist;
c) die Menge an mtDNA in der Probe, welche die Deletion aufweist, mit mindestens einem bekannten Referenzwert vergleicht;
wobei die Krebserkrankung Brustkrebs ist.

2. Das Verfahren nach Anspruch 1, wobei der mindestens eine bekannte Referenzwert die Menge der Deletion in einer mtDNA-Referenzprobe aus bekanntem, nicht karzinomatösem Gewebe oder bekannter, nicht karzinomatöser Körperflüssigkeit ist.

3. Das Verfahren nach Anspruch 1, wobei der mindestens eine bekannte Referenzwert die Menge der Deletion in einer mtDNA-Referenzprobe aus bekanntem, karzinomatösem Gewebe oder bekannter, karzinomatöser Körperflüssigkeit ist.

4. Das Verfahren nach Anspruch 1, wobei der bekannte Referenzwert die Menge der Deletion in einer mtDNAReferenzprobe aus bekanntem, nicht karzinomatösem Gewebe oder bekannter, nicht karzinomatöser Körperflüssigkeit ist,
wobei eine erhöhte Deletionsmenge in der biologischen Probe im Vergleich zu der Referenzprobe eine Krebserkrankung anzeigt.

5. Das Verfahren nach Anspruch 4, ferner einen Schritt umfassend, bei dem man die Menge an mtDNA in der Probe, welche die Deletion aufweist, mit der Menge der Deletion in einer mtDNA-Referenzprobe aus bekanntem, karzinomatösem Gewebe oder bekannter, karzinomatöser Körperflüssigkeit vergleicht.

6. Das Verfahren nach Anspruch 1, wobei der bekannte Referenzwert die Menge der Deletion in einer mtDNAReferenzprobe aus bekanntem, karzinomatösem Gewebe oder bekannter, karzinomatöser Körperflüssigkeit ist,
wobei ein ähnliches Niveau der Deletion in der biologischen Probe im Vergleich zu der Referenzprobe eine Krebserkrankung anzeigt.

7. Das Verfahren nach Anspruch 6, ferner einen Schritt umfassend, bei dem man die Menge an mtDNA in der Probe, welche die Deletion aufweist, mit der Menge der Deletion in einer mtDNA-Referenzprobe aus bekanntem, nicht karzinomatösem Gewebe oder bekannter, nicht karzinomatöser Körperflüssigkeit vergleicht.

8. Verfahren zur Überwachung einer Person auf die Entwicklung einer Krebserkrankung, Schritte umfassend, bei denen man:
a) mtDNA aus einer biologischen Probe, die aus der Person stammt, extrahiert;
b) die Menge an mtDNA in der Probe quantifiziert, die eine Deletion von ungefähr 3379 Basenpaaren in der Nukleinsäuresequenz, die ungefähr die Positionen 10744 bis 14124 des mtDNA-Genoms umfasst, aufweist;
c) Schritte a) bis b) über eine Zeitspanne wiederholt,
wobei ein erhöhtes Niveau der Deletion über die Zeitspanne eine Krebserkrankung anzeigt;
wobei die Krebserkrankung Brustkrebs ist.

9. Verfahren nach Anspruch 8, ferner mindestens einen Schritt umfassend, der ausgewählt ist aus der Gruppe bestehend aus:
(a) Vergleichen der Menge der mtDNA in der Probe, welche die Deletion aufweist, mit der Menge der Deletion in einer mtDNA-Referenzprobe aus bekanntem, nicht karzinomatösen Gewebe oder bekannter, nicht karzinomatöser Körperflüssigkeit; und
(b) Vergleichen der Menge der mtDNA in der Probe, welche die Deletion aufweist, mit der Menge der Deletion in einer mtDNA-Referenzprobe aus bekanntem, karzinomatösen Gewebe oder bekannter, karzinomatöser Körperflüssigkeit.

10. Verfahren nach einem der vorangehenden Ansprüche, wobei die Deletion eine Nukleinsäuresequenz aufweist, die der in SEQ ID NO:1 gezeigten entspricht.

11. Verfahren nach einem der vorangehenden Ansprüche, wobei die Quantifizierung der Deletion zunächst die Amplifizierung einer mtDNA-Zielregion, welche die Deletion anzeigt, und die Quantifizierung der Menge der amplifizierten Zielregion umfasst.

12. Verfahren nach Anspruch 11, wobei die Amplifizierung der Zielregion unter Verwendung eines Paares von Amplifizierungsprimern durchgeführt wird, wobei eines der Paare von Amplifizierungsprimern mit einer Verbindungsstelle überlappt, welche die Abschnitte entgegengesetzter Enden der Deletion verbindet.

13. Verfahren nach einem der vorangehenden Ansprüche, wobei der Quantifizierungsschritt unter Verwendung von Echtzeit-PCR durchgeführt wird.

14. Verfahren nach Anspruch 1, wobei der Quantifizierungsschritt unter Verwendung von Echtzeit-PCR durchgeführt wird und wobei der Referenzwert ein Zyklusschwellenwert ist.

15. Verfahren nach einem der Ansprüche 11 oder 14, wobei ein PCR Primer als Teil eines Paares von Amplifikationsprimern zur Amplifizierung der Zielregion verwendet wird, der eine Sequenz aufweist, die der von SEQ ID NO:2 entspricht.

16. Verfahren nach einem der Ansprüche 11 oder 14, wobei einer eines Paares von PCR Primern, die bei der Amplifizierung der Zielregion verwendet werden, nach der Deletion der Sequenz, die der von SEQ ID NO:1 entspricht, einen gespleißten mtDNA-Abschnitt überlappt.

17. Verfahren nach einem der vorangehenden Ansprüche, wobei die biologische Probe ein Körpergewebe oder eine Körperflüssigkeit ist.

18. Verfahren nach Anspruch 17, wobei die biologische Probe aus Brustgewebe und Urin ausgewählt ist.

## Revendications

1. Procédé pour détecter un cancer chez un individu comprenant :
a) l'extraction de l'ADN mitochondrial, ADNmt, à partir d'un échantillon biologique provenant de l'individu ;
b) la quantification de la quantité d'ADNmt dans l'échantillon ayant une délétion d'approximativement 3379 paires de bases dans la séquence d'acides nucléiques s'étendant approximativement des résidus 10 744 à 14 124 du génome d'ADNmt ;
c) la comparaison de la quantité d'ADNmt dans l'échantillon ayant la délétion avec au moins une valeur de référence connue ;
dans lequel le cancer est le cancer du sein.

2. Procédé selon la revendication 1, dans lequel l'au moins une valeur de référence connue est la quantité de délétions dans un échantillon de référence d'ADNmt provenant d'un tissu ou d'un fluide corporel non cancéreux connu.

3. Procédé selon la revendication 1, dans lequel l'au moins une valeur de référence connue est la quantité de délétions dans un échantillon de référence d'ADNmt provenant d'un tissu ou d'un fluide corporel cancéreux connu.

4. Procédé selon la revendication 1, dans lequel la valeur de référence connue est la quantité de délétions dans un échantillon de référence d'ADNmt provenant d'un tissu ou d'un fluide corporel non cancéreux connu,
dans lequel une quantité élevée de délétions dans l'échantillon biologique comparé à l'échantillon de référence indique un cancer.

5. Procédé selon la revendication 4, comprenant en outre l'étape consistant à comparer la quantité d'ADNmt dans l'échantillon ayant la délétion avec la quantité de délétions dans un échantillon de référence d'ADNmt provenant d'un tissu ou d'un fluide corporel cancéreux connu.

6. Procédé selon la revendication 1, dans lequel la valeur de référence connue est la quantité de délétions dans un échantillon de référence d'ADNmt provenant d'un tissu ou d'un fluide corporel cancéreux connu,
dans lequel un niveau similaire de délétion dans l'échantillon biologique comparé à l'échantillon de référence indique un cancer.

7. Procédé selon la revendication 6, comprenant en outre l'étape consistant à comparer la quantité d'ADNmt dans l'échantillon ayant la délétion avec la quantité de délétions dans un échantillon de référence d'ADNmt provenant d'un tissu ou d'un fluide corporel non cancéreux connu.

8. Procédé destiné à surveiller chez un individu le développement d'un cancer comprenant :
a) l'extraction de l'ADNmt à partir d'un échantillon biologique provenant de l'individu ;
b) la quantification de la quantité d'ADNmt dans l'échantillon ayant une délétion d'approximativement 3379 paires de bases dans la séquence d'acides nucléiques s'étendant approximativement des résidus 10 744 à 14 124 du génome d'ADNmt ;
c) la répétition des étapes a) à b) pendant une période de temps,
dans lequel un niveau croissant de la délétion pendant la période de temps indique un cancer ;
dans lequel cancer est le cancer du sein.

9. Procédé selon la revendication 8, comprenant en outre au moins une étape choisie parmi le groupe comprenant : (a) la comparaison de la quantité d'ADNmt dans l'échantillon ayant la délétion avec la quantité de délétions dans un échantillon de référence d'ADNmt provenant d'un tissu ou d'un fluide corporel non cancéreux connu ; et (b) la comparaison de la quantité d'ADNmt dans l'échantillon ayant la délétion avec la quantité de délétions dans un échantillon de référence d'ADNmt provenant d'un tissu ou d'un fluide corporel cancéreux connu.

10. Procédé selon l'une quelconque des revendications précédentes, dans lequel la délétion présente une séquence d'acides nucléiques correspondant à la séquence identifiée dans la SEQ ID NO : 1.

11. Procédé selon l'une quelconque des revendications précédentes, dans lequel la quantification de la délétion consiste à d'abord amplifier une région cible de l'ADNmt qui indique la délétion, et à quantifier la quantité de la région cible amplifiée.

12. Procédé selon la revendication 11, dans lequel l'amplification de la région cible est réalisée en utilisant une paire d'amorces d'amplification, une amorce de la paire d'amorces d'amplification recouvrant des régions de jonction d'épissage aux extrémités opposées de la délétion.

13. Procédé selon l'une quelconque des revendications précédentes, dans lequel l'étape de quantification est réalisée en utilisant une PCR en temps réel.

14. Procédé selon la revendication 1, dans lequel l'étape de quantification est réalisée en utilisant une PCR en temps réel et dans lequel la valeur de référence est un seuil de cycle.

15. Procédé selon la revendication 11 ou 14, dans lequel une amorce de PCR ayant une séquence correspondant à la SEQ ID NO : 2 est utilisée en tant qu'une partie d'une paire d'amorces d'amplification pour amplifier la région cible.

16. Procédé selon la revendication 11 ou 14, dans lequel une amorce d'une paire d'amorces de PCR utilisée dans l'amplification de la région cible recouvre une région épissée d'ADNmt après délétion de la séquence correspondant à la SEQ ID NO : 1.

17. Procédé selon l'une quelconque des revendications précédentes, dans lequel l'échantillon biologique est un tissu corporel ou un fluide corporel.

18. Procédé selon la revendication 17, dans lequel l'échantillon biologique est choisi parmi du tissu mammaire et de l'urine.


### SEQUENCE LISTING

<110> Genesis Genomics Inc.
<120> 3.4 KB Mitochondrial DNA Deletion For Use in The Detection of Cancer
<130> 102222/00031
<160> 10
<170> PatentIn version 3.3
<210> 1
   <211> 3379
   <212> DNA
   <213> Homo sapiens
<400> 1 ctttcttctt cccactcat 3379
<210> 2
   <211> 30
   <212> DNA
   <213> artificial
<220>
   <223> 3.4 kb deletion forward detection primer
<400> 2
   tagactacgt acatactaac cctactccta 30
<210> 3
   <211> 19
   <212> DNA
   <213> artificial
<220>
   <223> 3.4 mtDNA deletion reverse detection primer
<400> 3
   gaggtaggat tggtgctgt 19
<210> 4
   <211> 21
   <212> DNA
   <213> artificial
<220>
   <223> mtDNA genome forward primer
<400> 4
   cgttccagtg agttcaccct c 21
<210> 5
   <211> 23
   <212> DNA
   <213> artificial
<220>
   <223> mtDNA genome reverse primer
<400> 5
   cactctttac gccggcttct att 23
<210> 6
   <211> 20
   <212> DNA
   <213> artificial
<220>
   <223> TNF nuclear gene forward primer
<400> 6
   cctgccccaa tccctttatt 20
<210> 7
   <211> 21
   <212> DNA
   <213> artificial
<220>
   <223> TNF nuclear gene reverse primer
<400> 7
   ggtttcgaag tggtggtctt g 21
<210> 8
   <211> 16569
   <212> DNA
   <213> homo sapiens
<220>
   <221> misc_feature
   <222> (3107)..(3107)
   <223> n is a, c, g, or t
<300>
   <308> AC_000021
   <309> 2007-03-07
   <313> (1)..(16569)
<400> 8
   gatcacaggt ctatcaccct attaaccact cacgggagct ctccatgcat ttggtatttt     60
   cgtctggggg gtatgcacgc gatagcattg cgagacgctg gagccggagc accctatgtc    120
   gcagtatctg tctttgattc ctgcctcatc ctattattta tcgcacctac gttcaatatt    180
   acaggcgaac atacttacta aagtgtgtta attaattaat gcttgtagga cataataata    240
   acaattgaat gtctgcacag ccactttcca cacagacatc ataacaaaaa atttccacca    300
   aaccccccct cccccgcttc tggccacagc acttaaacac atctctgcca aaccccaaaa    360
   acaaagaacc ctaacaccag cctaaccaga tttcaaattt tatcttttgg cggtatgcac    420
   ttttaacagt caccccccaa ctaacacatt attttcccct cccactccca tactactaat    480
   ctcatcaata caacccccgc ccatcctacc cagcacacac acaccgctgc taaccccata    540
   ccccgaacca accaaacccc aaagacaccc cccacagttt atgtagctta cctcctcaaa    600
   gcaatacact gaaaatgttt agacgggctc acatcacccc ataaacaaat aggtttggtc    660
   ctagcctttc tattagctct tagtaagatt acacatgcaa gcatccccgt tccagtgagt    720
   tcaccctcta aatcaccacg atcaaaagga acaagcatca agcacgcagc aatgcagctc    780
   aaaacgctta gcctagccac acccccacgg gaaacagcag tgattaacct ttagcaataa    840
   acgaaagttt aactaagcta tactaacccc agggttggtc aatttcgtgc cagccaccgc    900
   ggtcacacga ttaacccaag tcaatagaag ccggcgtaaa gagtgtttta gatcaccccc    960
   tccccaataa agctaaaact cacctgagtt gtaaaaaact ccagttgaca caaaatagac   1020
   tacgaaagtg gctttaacat atctgaacac acaatagcta agacccaaac tgggattaga   1080
   taccccacta tgcttagccc taaacctcaa cagttaaatc aacaaaactg ctcgccagaa   1140
   cactacgagc cacagcttaa aactcaaagg acctggcggt gcttcatatc cctctagagg 1200
   agcctgttct gtaatcgata aaccccgatc aacctcacca cctcttgctc agcctatata 1260
   ccgccatctt cagcaaaccc tgatgaaggc tacaaagtaa gcgcaagtac ccacgtaaag 1320
   acgttaggtc aaggtgtagc ccatgaggtg gcaagaaatg ggctacattt tctaccccag 1380
   aaaactacga tagcccttat gaaacttaag ggtcgaaggt ggatttagca gtaaactaag 1440
   agtagagtgc ttagttgaac agggccctga agcgcgtaca caccgcccgt caccctcctc 1500
   aagtatactt caaaggacat ttaactaaaa cccctacgca tttatataga ggagacaagt 1560
   cgtaacatgg taagtgtact ggaaagtgca cttggacgaa ccagagtgta gcttaacaca 1620
   aagcacccaa cttacactta ggagatttca acttaacttg accgctctga gctaaaccta 1680
   gccccaaacc cactccacct tactaccaga caaccttagc caaaccattt acccaaataa 1740
   agtataggcg atagaaattg aaacctggcg caatagatat agtaccgcaa gggaaagatg 1800
   aaaaattata accaagcata atatagcaag gactaacccc tataccttct gcataatgaa 1860
   ttaactagaa ataactttgc aaggagagcc aaagctaaga cccccgaaac cagacgagct 1920
   acctaagaac agctaaaaga gcacacccgt ctatgtagca aaatagtggg aagatttata 1980
   ggtagaggcg acaaacctac cgagcctggt gatagctggt tgtccaagat agaatcttag 2040
   ttcaacttta aatttgccca cagaaccctc taaatcccct tgtaaattta actgttagtc 2100
   caaagaggaa cagctctttg gacactagga aaaaaccttg tagagagagt aaaaaattta 2160
   acacccatag taggcctaaa agcagccacc aattaagaaa gcgttcaagc tcaacaccca 2220
   ctacctaaaa aatcccaaac atataactga actcctcaca cccaattgga ccaatctatc 2280
   accctataga agaactaatg ttagtataag taacatgaaa acattctcct ccgcataagc 2340
   ctgcgtcaga ttaaaacact gaactgacaa ttaacagccc aatatctaca atcaaccaac 2400
   aagtcattat taccctcact gtcaacccaa cacaggcatg ctcataagga aaggttaaaa 2460
   aaagtaaaag gaactcggca aatcttaccc cgcctgttta ccaaaaacat cacctctagc 2520
   atcaccagta ttagaggcac cgcctgccca gtgacacatg tttaacggcc gcggtaccct 2580
   aaccgtgcaa aggtagcata atcacttgtt ccttaaatag ggacctgtat gaatggctcc 2640
   acgagggttc agctgtctct tacttttaac cagtgaaatt gacctgcccg tgaagaggcg 2700
   ggcataacac agcaagacga gaagacccta tggagcttta atttattaat gcaaacagta 2760
   cctaacaaac ccacaggtcc taaactacca aacctgcatt aaaaatttcg gttggggcga 2820
   cctcggagca gaacccaacc tccgagcagt acatgctaag acttcaccag tcaaagcgaa 2880
   ctactatact caattgatcc aataacttga ccaacggaac aagttaccct agggataaca 2940
   gcgcaatcct attctagagt ccatatcaac aatagggttt acgacctcga tgttggatca 3000
   ggacatcccg atggtgcagc cgctattaaa ggttcgtttg ttcaacgatt aaagtcctac 3060
   gtgatctgag ttcagaccgg agtaatccag gtcggtttct atctacnttc aaattcctcc 3120
   ctgtacgaaa ggacaagaga aataaggcct acttcacaaa gcgccttccc ccgtaaatga 3180
   tatcatctca acttagtatt atacccacac ccacccaaga acagggtttg ttaagatggc 3240
   agagcccggt aatcgcataa aacttaaaac tttacagtca gaggttcaat tcctcttctt 3300
   aacaacatac ccatggccaa cctcctactc ctcattgtac ccattctaat cgcaatggca 3360
   ttcctaatgc ttaccgaacg aaaaattcta ggctatatac aactacgcaa aggccccaac 3420
   gttgtaggcc cctacgggct actacaaccc ttcgctgacg ccataaaact cttcaccaaa 3480
   gagcccctaa aacccgccac atctaccatc accctctaca tcaccgcccc gaccttagct 3540
   ctcaccatcg ctcttctact atgaaccccc ctccccatac ccaaccccct ggtcaacctc 3600
   aacctaggcc tcctatttat tctagccacc tctagcctag ccgtttactc aatcctctga 3660
   tcagggtgag catcaaactc aaactacgcc ctgatcggcg cactgcgagc agtagcccaa 3720
   acaatctcat atgaagtcac cctagccatc attctactat caacattact aataagtggc 3780
   tcctttaacc tctccaccct tatcacaaca caagaacacc tctgattact cctgccatca 3840
   tgacccttgg ccataatatg atttatctcc acactagcag agaccaaccg aacccccttc 3900
   gaccttgccg aaggggagtc cgaactagtc tcaggcttca acatcgaata cgccgcaggc 3960
   cccttcgccc tattcttcat agccgaatac acaaacatta ttataataaa caccctcacc 4020
   actacaatct tcctaggaac aacatatgac gcactctccc ctgaactcta cacaacatat 4080
   tttgtcacca agaccctact tctaacctcc ctgttcttat gaattcgaac agcatacccc 4140
   cgattccgct acgaccaact catacacctc ctatgaaaaa acttcctacc actcacccta 4200
   gcattactta tatgatatgt ctccataccc attacaatct ccagcattcc ccctcaaacc 4260
   taagaaatat gtctgataaa agagttactt tgatagagta aataatagga gcttaaaccc 4320
   ccttatttct aggactatga gaatcgaacc catccctgag aatccaaaat tctccgtgcc 4380
   acctatcaca ccccatccta aagtaaggtc agctaaataa gctatcgggc ccataccccg 4440
   aaaatgttgg ttataccctt cccgtactaa ttaatcccct ggcccaaccc gtcatctact 4500
   ctaccatctt tgcaggcaca ctcatcacag cgctaagctc gcactgattt tttacctgag 4560
   taggcctaga aataaacatg ctagctttta ttccagttct aaccaaaaaa ataaaccctc 4620
   gttccacaga agctgccatc aagtatttcc tcacgcaagc aaccgcatcc ataatccttc 4680
   taatagctat cctcttcaac aatatactct ccggacaatg aaccataacc aatactacca 4740
   atcaatactc atcattaata atcataatag ctatagcaat aaaactagga atagccccct 4800
   ttcacttctg agtcccagag gttacccaag gcacccctct gacatccggc ctgcttcttc 4860
   tcacatgaca aaaactagcc cccatctcaa tcatatacca aatctctccc tcactaaacg 4920
   taagccttct cctcactctc tcaatcttat ccatcatagc aggcagttga ggtggattaa 4980
   accaaaccca gctacgcaaa atcttagcat actcctcaat tacccacata ggatgaataa 5040
   tagcagttct accgtacaac cctaacataa ccattcttaa tttaactatt tatattatcc 5100
   taactactac cgcattccta ctactcaact taaactccag caccacgacc ctactactat   5160
   ctcgcacctg aaacaagcta acatgactaa cacccttaat tccatccacc ctcctctccc   5220
   taggaggcct gcccccgcta accggctttt tgcccaaatg ggccattatc gaagaattca 5280
   caaaaaacaa tagcctcatc atccccacca tcatagccac catcaccctc cttaacctct 5340
   acttctacct acgcctaatc tactccacct caatcacact actccccata tctaacaacg 5400
   taaaaataaa atgacagttt gaacatacaa aacccacccc attcctcccc acactcatcg 5460
   cccttaccac gctactccta cctatctccc cttttatact aataatctta tagaaattta 5520
   ggttaaatac agaccaagag ccttcaaagc cctcagtaag ttgcaatact taatttctgt 5580
   aacagctaag gactgcaaaa ccccactctg catcaactga acgcaaatca gccactttaa 5640
   ttaagctaag cccttactag accaatggga cttaaaccca caaacactta gttaacagct 5700
   aagcacccta atcaactggc ttcaatctac ttctcccgcc gccgggaaaa aaggcgggag 5760
   aagccccggc aggtttgaag ctgcttcttc gaatttgcaa ttcaatatga aaatcacctc 5820
   ggagctggta aaaagaggcc taacccctgt ctttagattt acagtccaat gcttcactca 5880
   gccattttac ctcaccccca ctgatgttcg ccgaccgttg actattctct acaaaccaca 5940
   aagacattgg aacactatac ctattattcg gcgcatgagc tggagtccta ggcacagctc 6000
   taagcctcct tattcgagcc gagctgggcc agccaggcaa ccttctaggt aacgaccaca 6060
   tctacaacgt tatcgtcaca gcccatgcat ttgtaataat cttcttcata gtaataccca 6120
   tcataatcgg aggctttggc aactgactag ttcccctaat aatcggtgcc cccgatatgg 6180
   cgtttccccg cataaacaac ataagcttct gactcttacc tccctctctc ctactcctgc 6240
   tcgcatctgc tatagtggag gccggagcag gaacaggttg aacagtctac cctcccttag 6300
   cagggaacta ctcccaccct ggagcctccg tagacctaac catcttctcc ttacacctag 6360
   caggtgtctc ctctatctta ggggccatca atttcatcac aacaattatc aatataaaac 6420
   cccctgccat aacccaatac caaacgcccc tcttcgtctg atccgtccta atcacagcag 6480
   tcctacttct cctatctctc ccagtcctag ctgctggcat cactatacta ctaacagacc 6540
   gcaacctcaa caccaccttc ttcgaccccg ccggaggagg agaccccatt ctataccaac 6600
   acctattctg atttttcggt caccctgaag tttatattct tatcctacca ggcttcggaa 6660
   taatctccca tattgtaact tactactccg gaaaaaaaga accatttgga tacataggta 6720
   tggtctgagc tatgatatca attggcttcc tagggtttat cgtgtgagca caccatatat 6780
   ttacagtagg aatagacgta gacacacgag catatttcac ctccgctacc ataatcatcg 6840
   ctatccccac cggcgtcaaa gtatttagct gactcgccac actccacgga agcaatatga 6900
   aatgatctgc tgcagtgctc tgagccctag gattcatctt tcttttcacc gtaggtggcc 6960
   tgactggcat tgtattagca aactcatcac tagacatcgt actacacgac acgtactacg 7020
   ttgtagccca cttccactat gtcctatcaa taggagctgt atttgccatc ataggaggct   7080
   tcattcactg atttccccta ttctcaggct acaccctaga ccaaacctac gccaaaatcc   7140
   atttcactat catattcatc ggcgtaaatc taactttctt cccacaacac tttctcggcc   7200
   tatccggaat gccccgacgt tactcggact accccgatgc atacaccaca tgaaacatcc   7260
   tatcatctgt aggctcattc atttctctaa cagcagtaat attaataatt ttcatgattt   7320
   gagaagcctt cgcttcgaag cgaaaagtcc taatagtaga agaaccctcc ataaacctgg   7380
   agtgactata tggatgcccc ccaccctacc acacattcga agaacccgta tacataaaat   7440
   ctagacaaaa aaggaaggaa tcgaaccccc caaagctggt ttcaagccaa ccccatggcc   7500
   tccatgactt tttcaaaaag gtattagaaa aaccatttca taactttgtc aaagttaaat   7560
   tataggctaa atcctatata tcttaatggc acatgcagcg caagtaggtc tacaagacgc   7620
   tacttcccct atcatagaag agcttatcac ctttcatgat cacgccctca taatcatttt   7680
   ccttatctgc ttcctagtcc tgtatgccct tttcctaaca ctcacaacaa aactaactaa   7740
   tactaacatc tcagacgctc aggaaataga aaccgtctga actatcctgc ccgccatcat   7800
   cctagtcctc atcgccctcc catccctacg catcctttac ataacagacg aggtcaacga   7860
   tccctccctt accatcaaat caattggcca ccaatggtac tgaacctacg agtacaccga   7920
   ctacggcgga ctaatcttca actcctacat acttccccca ttattcctag aaccaggcga   7980
   cctgcgactc cttgacgttg acaatcgagt agtactcccg attgaagccc ccattcgtat   8040
   aataattaca tcacaagacg tcttgcactc atgagctgtc cccacattag gcttaaaaac   8100
   agatgcaatt cccggacgtc taaaccaaac cactttcacc gctacacgac cgggggtata    8160
   ctacggtcaa tgctctgaaa tctgtggagc aaaccacagt ttcatgccca tcgtcctaga   8220
   attaattccc ctaaaaatct ttgaaatagg gcccgtattt accctatagc accccctcta   8280
   ccccctctag agcccactgt aaagctaact tagcattaac cttttaagtt aaagattaag   8340
   agaaccaaca cctctttaca gtgaaatgcc ccaactaaat actaccgtat ggcccaccat   8400
   aattaccccc atactcctta cactattcct catcacccaa ctaaaaatat taaacacaaa   8460
   ctaccaccta cctccctcac caaagcccat aaaaataaaa aattataaca aaccctgaga   8520
   accaaaatga acgaaaatct gttcgcttca ttcattgccc ccacaatcct aggcctaccc   8580
   gccgcagtac tgatcattct atttccccct ctattgatcc ccacctccaa atatctcatc   8640
   aacaaccgac taatcaccac ccaacaatga ctaatcaaac taacctcaaa acaaatgata   8700
   accatacaca acactaaagg acgaacctga tctcttatac tagtatcctt aatcattttt   8760
   attgccacaa ctaacctcct cggactcctg cctcactcat ttacaccaac cacccaacta   8820
   tctataaacc tagccatggc catcccctta tgagcgggca cagtgattat aggctttcgc   8880
   tctaagatta aaaatgccct agcccacttc ttaccacaag gcacacctac accccttatc   8940
   cccatactag ttattatcga aaccatcagc ctactcattc aaccaatagc cctggccgta 9000
   cgcctaaccg ctaacattac tgcaggccac ctactcatgc acctaattgg aagcgccacc 9060
   ctagcaatat caaccattaa ccttccctct acacttatca tcttcacaat tctaattcta 9120
   ctgactatcc tagaaatcgc tgtcgcctta atccaagcct acgttttcac acttctagta 9180
   agcctctacc tgcacgacaa cacataatga cccaccaatc acatgcctat catatagtaa 9240
   aacccagccc atgaccccta acaggggccc tctcagccct cctaatgacc tccggcctag 9300
   ccatgtgatt tcacttccac tccataacgc tcctcatact aggcctacta accaacacac 9360
   taaccatata ccaatgatgg cgcgatgtaa cacgagaaag cacataccaa ggccaccaca 9420
   caccacctgt ccaaaaaggc cttcgatacg ggataatcct atttattacc tcagaagttt 9480
   ttttcttcgc aggatttttc tgagcctttt accactccag cctagcccct accccccaat 9540
   taggagggca ctggccccca acaggcatca ccccgctaaa tcccctagaa gtcccactcc 9600
   taaacacatc cgtattactc gcatcaggag tatcaatcac ctgagctcac catagtctaa 9660
   tagaaaacaa ccgaaaccaa ataattcaag cactgcttat tacaatttta ctgggtctct 9720
   attttaccct cctacaagcc tcagagtact tcgagtctcc cttcaccatt tccgacggca 9780
   tctacggctc aacatttttt gtagccacag gcttccacgg acttcacgtc attattggct 9840
   caactttcct cactatctgc ttcatccgcc aactaatatt tcactttaca tccaaacatc 9900
   actttggctt cgaagccgcc gcctgatact ggcattttgt agatgtggtt tgactatttc 9960
   tgtatgtctc catctattga tgagggtctt actcttttag tataaatagt accgttaact 10020
   tccaattaac tagttttgac aacattcaaa aaagagtaat aaacttcgcc ttaattttaa 10080
   taatcaacac cctcctagcc ttactactaa taattattac attttgacta ccacaactca 10140
   acggctacat agaaaaatcc accccttacg agtgcggctt cgaccctata tcccccgccc 10200
   gcgtcccttt ctccataaaa ttcttcttag tagctattac cttcttatta tttgatctag 10260
   aaattgccct ccttttaccc ctaccatgag ccctacaaac aactaacctg ccactaatag 10320
   ttatgtcatc cctcttatta atcatcatcc tagccctaag tctggcctat gagtgactac 10380
   aaaaaggatt agactgaacc gaattggtat atagtttaaa caaaacgaat gatttcgact 10440
   cattaaatta tgataatcat atttaccaaa tgcccctcat ttacataaat attatactag 10500
   catttaccat ctcacttcta ggaatactag tatatcgctc acacctcata tcctccctac 10560
   tatgcctaga aggaataata ctatcgctgt tcattatagc tactctcata accctcaaca 10620
   cccactccct cttagccaat attgtgccta ttgccatact agtctttgcc gcctgcgaag 10680
   cagcggtggg cctagcccta ctagtctcaa tctccaacac atatggccta gactacgtac 10740
   ataacctaaa cctactccaa tgctaaaact aatcgtccca acaattatat tactaccact 10800
   gacatgactt tccaaaaaac acataatttg aatcaacaca accacccaca gcctaattat 10860
   tagcatcatc cctctactat tttttaacca aatcaacaac aacctattta gctgttcccc 10920
   aaccttttcc tccgaccccc taacaacccc cctcctaata ctaactacct gactcctacc 10980
   cctcacaatc atggcaagcc aacgccactt atccagtgaa ccactatcac gaaaaaaact 11040
   ctacctctct atactaatct ccctacaaat ctccttaatt ataacattca cagccacaga 11100
   actaatcata ttttatatct tcttcgaaac cacacttatc cccaccttgg ctatcatcac 11160
   ccgatgaggc aaccagccag aacgcctgaa cgcaggcaca tacttcctat tctacaccct 11220
   agtaggctcc cttcccctac tcatcgcact aatttacact cacaacaccc taggctcact 11280
   aaacattcta ctactcactc tcactgccca agaactatca aactcctgag ccaacaactt 11340
   aatatgacta gcttacacaa tagcttttat agtaaagata cctctttacg gactccactt 11400
   atgactccct aaagcccatg tcgaagcccc catcgctggg tcaatagtac ttgccgcagt 11460
   actcttaaaa ctaggcggct atggtataat acgcctcaca ctcattctca accccctgac 11520
   aaaacacata gcctacccct tccttgtact atccctatga ggcataatta taacaagctc 11580
   catctgccta cgacaaacag acctaaaatc gctcattgca tactcttcaa tcagccacat 11640
   agccctcgta gtaacagcca ttctcatcca aaccccctga agcttcaccg gcgcagtcat 11700
   tctcataatc gcccacgggc ttacatcctc attactattc tgcctagcaa actcaaacta 11760
   cgaacgcact cacagtcgca tcataatcct ctctcaagga cttcaaactc tactcccact 11820
   aatagctttt tgatgacttc tagcaagcct cgctaacctc gccttacccc ccactattaa 11880
   cctactggga gaactctctg tgctagtaac cacgttctcc tgatcaaata tcactctcct 11940
   acttacagga ctcaacatac tagtcacagc cctatactcc ctctacatat ttaccacaac 12000
   acaatggggc tcactcaccc accacattaa caacataaaa ccctcattca cacgagaaaa 12060
   caccctcatg ttcatacacc tatcccccat tctcctccta tccctcaacc ccgacatcat 12120
   taccgggttt tcctcttgta aatatagttt aaccaaaaca tcagattgtg aatctgacaa 12180
   cagaggctta cgacccctta tttaccgaga aagctcacaa gaactgctaa ctcatgcccc 12240
   catgtctaac aacatggctt tctcaacttt taaaggataa cagctatcca ttggtcttag 12300
   gccccaaaaa ttttggtgca actccaaata aaagtaataa ccatgcacac tactataacc 12360
   accctaaccc tgacttccct aattcccccc atccttacca ccctcgttaa ccctaacaaa 12420
   aaaaactcat acccccatta tgtaaaatcc attgtcgcat ccacctttat tatcagtctc 12480
   ttccccacaa caatattcat gtgcctagac caagaagtta ttatctcgaa ctgacactga 12540
   gccacaaccc aaacaaccca gctctcccta agcttcaaac tagactactt ctccataata 12600
   ttcatccctg tagcattgtt cgttacatgg tccatcatag aattctcact gtgatatata 12660
   aactcagacc caaacattaa tcagttcttc aaatatctac tcatcttcct aattaccata 12720
   ctaatcttag ttaccgctaa caacctattc caactgttca tcggctgaga gggcgtagga 12780
   attatatcct tcttgctcat cagttgatga tacgcccgag cagatgccaa cacagcagcc 12840
   attcaagcaa tcctatacaa ccgtatcggc gatatcggtt tcatcctcgc cttagcatga 12900
   tttatcctac actccaactc atgagaccca caacaaatag cccttctaaa cgctaatcca 12960
   agcctcaccc cactactagg cctcctccta gcagcagcag gcaaatcagc ccaattaggt 13020
   ctccacccct gactcccctc agccatagaa ggccccaccc cagtctcagc cctactccac 13080
   tcaagcacta tagttgtagc aggaatcttc ttactcatcc gcttccaccc cctagcagaa 13140
   aatagcccac taatccaaac tctaacacta tgcttaggcg ctatcaccac tctgttcgca 13200
   gcagtctgcg cccttacaca aaatgacatc aaaaaaatcg tagccttctc cacttcaagt 13260
   caactaggac tcataatagt tacaatcggc atcaaccaac cacacctagc attcctgcac 13320
   atctgtaccc acgccttctt caaagccata ctatttatgt gctccgggtc catcatccac 13380
   aaccttaaca atgaacaaga tattcgaaaa ataggaggac tactcaaaac catacctctc 13440
   acttcaacct ccctcaccat tggcagccta gcattagcag gaataccttt cctcacaggt 13500
   ttctactcca aagaccacat catcgaaacc gcaaacatat catacacaaa cgcctgagcc 13560
   ctatctatta ctctcatcgc tacctccctg acaagcgcct atagcactcg aataattctt 13620
   ctcaccctaa caggtcaacc tcgcttcccc acccttacta acattaacga aaataacccc 13680
   accctactaa accccattaa acgcctggca gccggaagcc tattcgcagg atttctcatt  13740
   actaacaaca tttcccccgc atcccccttc caaacaacaa tccccctcta cctaaaactc  13800
   acagccctcg ctgtcacttt cctaggactt ctaacagccc tagacctcaa ctacctaacc 13860
   aacaaactta aaataaaatc cccactatgc acattttatt tctccaacat actcggattc 13920
   taccctagca tcacacaccg cacaatcccc tatctaggcc ttcttacgag ccaaaacctg 13980
   cccctactcc tcctagacct aacctgacta gaaaagctat tacctaaaac aatttcacag 14040
   caccaaatct ccacctccat catcacctca acccaaaaag gcataattaa actttacttc 14100
   ctctctttct tcttcccact catcctaacc ctactcctaa tcacataacc tattcccccg 14160
   agcaatctca attacaatat atacaccaac aaacaatgtt caaccagtaa ctactactaa 14220
   tcaacgccca taatcataca aagcccccgc accaatagga tcctcccgaa tcaaccctga 14280
   cccctctcct tcataaatta ttcagcttcc tacactatta aagtttacca caaccaccac 14340
   cccatcatac tctttcaccc acagcaccaa tcctacctcc atcgctaacc ccactaaaac 14400
   actcaccaag acctcaaccc ctgaccccca tgcctcagga tactcctcaa tagccatcgc 14460
   tgtagtatat ccaaagacaa ccatcattcc ccctaaataa attaaaaaaa ctattaaacc 14520
   catataacct cccccaaaat tcagaataat aacacacccg accacaccgc taacaatcaa 14580
   tactaaaccc ccataaatag gagaaggctt agaagaaaac cccacaaacc ccattactaa 14640
   acccacactc aacagaaaca aagcatacat cattattctc gcacggacta caaccacgac 14700
   caatgatatg aaaaaccatc gttgtatttc aactacaaga acaccaatga ccccaatacg 14760
   caaaactaac cccctaataa aattaattaa ccactcattc atcgacctcc ccaccccatc 14820
   caacatctcc gcatgatgaa acttcggctc actccttggc gcctgcctga tcctccaaat 14880
   caccacagga ctattcctag ccatgcacta ctcaccagac gcctcaaccg ccttttcatc 14940
   aatcgcccac atcactcgag acgtaaatta tggctgaatc atccgctacc ttcacgccaa 15000
   tggcgcctca atattcttta tctgcctctt cctacacatc gggcgaggcc tatattacgg 15060
   atcatttctc tactcagaaa cctgaaacat cggcattatc ctcctgcttg caactatagc 15120
   aacagccttc ataggctatg tcctcccgtg aggccaaata tcattctgag gggccacagt 15180
   aattacaaac ttactatccg ccatcccata cattgggaca gacctagttc aatgaatctg 15240
   aggaggctac tcagtagaca gtcccaccct cacacgattc tttacctttc acttcatctt 15300
   gcccttcatt attgcagccc tagcaacact ccacctccta ttcttgcacg aaacgggatc 15360
   aaacaacccc ctaggaatca cctcccattc cgataaaatc accttccacc cttactacac 15420
   aatcaaagac gccctcggct tacttctctt ccttctctcc ttaatgacat taacactatt 15480
   ctcaccagac ctcctaggcg acccagacaa ttatacccta gccaacccct taaacacccc 15540
   tccccacatc aagcccgaat gatatttcct attcgcctac acaattctcc gatccgtccc 15600
   taacaaacta ggaggcgtcc ttgccctatt actatccatc ctcatcctag caataatccc 15660
   catcctccat atatccaaac aacaaagcat aatatttcgc ccactaagcc aatcacttta 15720
   ttgactccta gccgcagacc tcctcattct aacctgaatc ggaggacaac cagtaagcta 15780
   cccttttacc atcattggac aagtagcatc cgtactatac ttcacaacaa tcctaatcct 15840
   aataccaact atctccctaa ttgaaaacaa aatactcaaa tgggcctgtc cttgtagtat 15900
   aaactaatac accagtcttg taaaccggag atgaaaacct ttttccaagg acaaatcaga 15960
   gaaaaagtct ttaactccac cattagcacc caaagctaag attctaattt aaactattct 16020
   ctgttctttc atggggaagc agatttgggt accacccaag tattgactca cccatcaaca 16080
   accgctatgt atttcgtaca ttactgccag ccaccatgaa tattgtacgg taccataaat 16140
   acttgaccac ctgtagtaca taaaaaccca atccacatca aaaccccctc cccatgctta 16200
   caagcaagta cagcaatcaa ccctcaacta tcacacatca actgcaactc caaagccacc 16260
   cctcacccac taggatacca acaaacctac ccacccttaa cagtacatag tacataaagc 16320
   catttaccgt acatagcaca ttacagtcaa atcccttctc gtccccatgg atgacccccc 16380
   tcagataggg gtcccttgac caccatcctc cgtgaaatca atatcccgca caagagtgct 16440
   actctcctcg ctccgggccc ataacacttg ggggtagcta aagtgaactg tatccgacat 16500
   ctggttccta cttcagggtc ataaagccta aatagcccac acgttcccct taaataagac 16560
   atcacgatg 16569
<210> 9
   <211> 22
   <212> DNA
   <213> artificial
<220>
   <223> Forward primer for detection of 3.4 kb mtDNA deleted sequence
<400> 9
   cccactcatc acctaaacct ac 22
<210> 10
   <211> 20
   <212> DNA
   <213> artificial
<220>
   <223> Reverse primer for 3.4 kb deleted sequence.
<400> 10
   ggtaggagtc aggtagttag 20